# EUROPEAN PATENT SPECIFICATION

(11) **EP 2 748 204 B1**
(45) Date of publication and mention of the grant of the patent: **20.04.2016**
(21) Application number: 12838521.8
(22) Date of filing: 03.10.2012
(51) Int. Cl.: C08B 37/00, C08L 5/00, A61K 31/715, A61P 31/04, A61P 31/10, C07H 15/12, C07H 15/18, A61K 31/7016, A61K 31/702, A61P 31/12, A61P 33/02

(54) **CATIONIC PEPTIDOPOLYSACCHARIDES WITH EXCELLENT BROAD- SPECTRUM ANTIMICROBIAL ACTIVITIES AND HIGH SELECTIVITY**
KATIONISCHE PEPTIDOPOLYSACCHARIDE MIT HERVORRAGENDEM BREITEM ANTIMIKROBIELLM WIRKUNGSSPEKTRUM UND HOHER SELEKTIVITÄT
PEPTIDOPOLYSACCHARIDES CATIONIQUES PRÉSENTANT D'EXCELLENTES ACTIVITÉS ANTIMICROBIENNES À LARGE SPECTRE ET UNE SÉLECTIVITÉ ÉLEVÉE

(30) Priority: 03.10.2011 US 201161542321 P
(43) Date of publication of application: 02.07.2014
(73) Proprietor: Nanyang Technological University, Singapore 639798 (SG)
(72) Inventor: CHAN, Bee Eng Mary, Singapore 639798 (SG); LI, Peng, Singapore 639798 (SG)
(74) Representative: Schiweck, Weinzierl & Koch
(86) International application number: PCT/SG2012/000371
(87) International publication number: WO 2013/052012

(56) References cited:
- EP-A1- 0 538 230
- EP-A2- 1 918 306
- WO-A1-2010/025542
- WO-A2-2009/037592
- KEIKO KONDO ET AL: "Study of a novel glycoconjugate, thiopeptidoglycan, and a novel polysaccharide lyase, thiopeptidoglycan lyase", INTERNATIONAL JOURNAL OF BIOLOGICAL MACROMOLECULES, ELSEVIER BV, NL, vol. 48, no. 2, 16 November 2010 (2010-11-16), pages 256-262, XP028360050, ISSN: 0141-8130, DOI: 10.1016/J.IJBIOMAC.2010.11.009 [retrieved on 2010-11-21]
- SPRENGARD, ULRICH ET AL.: 'Synthesis and biological activity of novel sialyl- LewisX conjugates' BIOORGANIC & MEDICINAL CHEMISTRY LETTERS vol. 6, no. 5, 05 March 1996, PERGAMON, ELSEVIER SCIENCE, GB, ISSN 0960-894X pages 509 - 14, XP004135018

## Description

### FIELD OF THE INVENTION

The present invention is in the field of microbiology, and more particularly, in the field of antimicrobial agents that kill or inhibit the growth of microorganisms such as bacteria, fungi and protozoa.

### BACKGROUND OF THE DISCLOSURE

The emergence of microbial resistance to conventional antibiotics is a serious challenge for human public health and has led to an overwhelming demand for new antimicrobial agents. Unlike conventional antibiotics that target microbe metabolism, natural antimicrobial peptides (AMPs) exert their antimicrobial activity by disrupting the cytoplasmic membrane of the microbe. This mode of antimicrobial activity is believed to be less susceptible to the development of resistance than metabolic targeting. Although natural AMPs are highly effective, their application is limited by their high toxicity, poor proteolytic stability and pharmacokinetics, and high cost of manufacture. Cationic AMPs and their synthetic analogues that also target the pathogen cytoplasmic membrane, provide a promising approach for lowering the likelihood of pathogen resistance. For all effective antimicrobial agents, the microbial cytoplasmic membrane is surrounded by a cell wall that is an inevitable barrier that needs to be penetrated. Previous designs of cationic antimicrobial polymers, however, generally failed to consider the structural affinity of the antimicrobial agents with the microbial cell wall. Therefore, there remains a need in the art for the design, synthesis and testing of new membrane-acting cationic AMPs and their analogues that are nontoxic, biocompatible and inexpensive to produce.

### SUMMARY OF THE INVENTION

The present invention addresses these needs by providing new and improved compounds and compositions for controlling, disinfecting and treating a microorganism. Also disclosed within the context of the present invention are methods for controlling, disinfecting and treating a microorganism.

Thus, in one embodiment the disclosure provides a compound of Formula I: 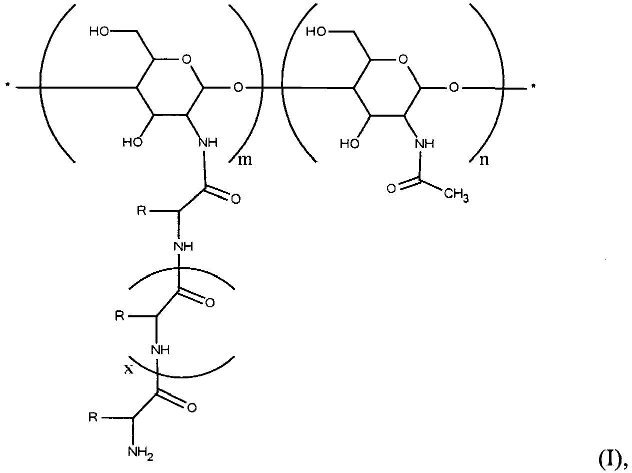 or a pharmaceutically acceptable salt thereof, wherein:
each R is independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, (CH₂)ⱼOR¹, (CH₂)ⱼC(O)R¹, (CH₂)ⱼC(O)OR¹, (CH₂)ⱼOC(O)R¹, (CH₂)ⱼNR²R³, (CH₂)ⱼC(O)NR²R³, (CH₂)ⱼOC(O)NR²R³, (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR ²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ and (CH₂)ⱼNR⁴S(O)ₖNR²R³, wherein j is an integer independently selected from 0 to 6, k is an integer independently selected from 0 to 2, and wherein each R group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
R¹, R², R³ and R⁴ are each independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, wherein each R¹, R², R³ and R⁴ group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
m is an integer independently selected from 1 to 100,000;
n is an integer independently selected from 1 to 100,000; and
x is an integer independently selected from 1 to 5,000.

In another embodiment the disclosure provides a pharmaceutical composition comprising a compound of Formula I and a pharmaceutically acceptable carrier.

In another embodiment the disclosure provides a therapeutic composition for controlling infection by a microorganism, including the compound of Formula I in a therapeutically effective amount and a pharmaceutically acceptable carrier.

Also disclosed within the context of the present invention are methods for controlling a microorganism by administering a therapeutically effective amount of a composition, wherein the composition includes the compound of Formula I and an acceptable carrier.

Also disclosed within the context of the present invention are methods for treating a subject in need of or preventing an infection in a subject caused by a microorganism by administering a therapeutically effective amount of a pharmaceutical composition of the compound of Formula I and a pharmaceutically acceptable carrier.

In another embodiment the disclosure provides ex-vivo methods for disinfecting a surface of an article by applying to the surface an effective amount of a composition, wherein the composition includes the compound of Formula I.

In another embodiment the disclosure provides a disinfecting solution including the compound of Formula I, and optionally an acceptable carrier.

In another embodiment the disclosure provides methods for preparing a compound of Formula I.

In yet another embodiment, the disclosure provides a compound of Formula I, prepared by the methods disclosed herein.

### DESCRIPTION OF THE DRAWINGS

Figure 1 illustrates the chemical structure of the cationic peptidopolysaccharide (chitosan-*graft*-polypeptide) (R = Lys, Phe, *etc.* side chain) and bacterial cell wall peptidoglycan (R = Ala, Glu, Lys, *etc*. side chain).
Figure 2 illustrates the proposed mode of action of the cationic peptidopolysaccharides on Gram-negative and Gram-positive bacteria.
Figure 3 illustrates the morphology of various pathogens cells before (left) and treated (right) with CS-*g*-K₁₆.
Figure 4 illustrates the LIVE/DEAD bacterial viability assay of: (a) *E. coli* control; and (b) *E. coli* treated with CS-*g*-K₁₆.
Figure 5 illustrates: (Fig.5a) Membrane potential sensitive dye DiSC₃(5) leakage from *E. coli* challenged with CS-*g*-K₁₆; and (Fig.5b) CD spectra of CS-*g*-K₁₆ in 20 mM sodium phosphate buffer with 0 µM, 5 µM and 500 µM small unilamellar liposomes (POPC/POPG 4:1).
Figure 6 illustrates the membrane potential sensitive dye DiSC₃(5) leakage from S. *aureus* challenged with CS-*g*-K₁₆.
Figure 7 illustrates the MTT of: (Fig.7a) SMCs cultured in a series of concentrations of CS-*g*-K₁₆ for 1 h (p > 0.05, no significant difference); (Fig.7b) SMCs cultured with 100 ug ml⁻¹ CS-*g*-K₁₆ from 1 to 5 days. Co-culture of SMC with *E. coli* after 6 h incubation: (Fig.7c) in the culture medium without antibiotic and CS-*g*-K₁₆, bacteria grew well but SMCs detached from the culture dish and change to round shape; (Fig.7d) in the culture medium with 100 µg ml⁻¹ CS-*g*-K₁₆, bacteria were inhibited and SMCs grew well.

### DETAILED DESCRIPTION OF EMBODIMENTS

### Definitions

Unless otherwise defined, scientific and technical terms used in connection with the disclosure shall have the meanings that are commonly understood by those of ordinary skill in the art. Further, unless otherwise required by context, singular terms shall include pluralities and plural terms shall include the singular. Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to manufacturer's specifications or as commonly accomplished in the art or as described herein. The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

The following terms, definitions and abbreviations further apply:

The term "alkyl," by itself or as part of another substituent, means, unless otherwise stated, a straight (i.e. unbranched) or branched chain, or cyclic hydrocarbon radical, or combination thereof, which may be fully saturated, mono- or polyunsaturated and can include di- and multivalent radicals, having the number of carbon atoms designated (i.e. C₁-C₁₀ means one to ten carbons). Examples of saturated hydrocarbon radicals include, but are not limited to, groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. Alkyl groups which are limited to hydrocarbon groups are termed "homoalkyl."

Specific values listed herein for groups, substituents, and ranges, are for illustration; they do not exclude other defined values or other values within defined ranges for the groups and substituents. For example, "alkyl" can be methyl, ethyl, propyl, isopropyl, butyl isobutyl, sec-butyl, pentyl, 3-pentyl, or hexyl; cycloalkyl can be cyclopropyl, cyclobutyl, cyclopentyl, or cyclohexyl; "-O(C₁-C₆)alkyl (alkoxy)" can be methoxy, ethoxy, propoxy, isopropoxy, butoxy, iso-butoxy, sec-butoxy, pentoxy, 3-pentoxy, or hexyloxy.

The term "alkylene" by itself or as part of another substituent means a divalent radical derived from an alkyl, as exemplified, but not limited, by -CH₂CH₂CH₂CH₂-, -CH₂CH=CHCH₂-, -CH₂CH=CCH₂-, -CH₂CH₂CH(CH₂CH₂CH₃)CH₂-. Typically, an alkyl (or alkylene) group will have from 1 to 24 carbon atoms, which includes those groups having 10 or fewer carbon atoms. A "lower alkyl" or "lower alkylene" is a shorter chain alkyl or alkylene group, generally having eight or fewer carbon atoms.

The terms "alkyl, alkoxy, alkenyl, alkynyl," etc. denote both straight and branched groups; but reference to an individual group such as "propyl" embraces the straight chain group, a branched chain isomer such as "isopropyl" being specifically referred to.

The term "heteroalkyl," by itself or in combination with another term, means, unless otherwise stated, a stable straight or branched chain, or cyclic hydrocarbon radical, or combinations thereof, consisting of at least one carbon atoms and at least one heteroatom selected from the group consisting of O, N, P, Si and S, and wherein the nitrogen, phosphorus, and sulfur atoms may optionally be oxidized and the nitrogen heteroatom may optionally be quaternized. The heteroatom(s) O, N, P and S and Si may be placed at any interior position of the heteroalkyl group or at the position at which alkyl group is attached to the remainder of the molecule. Examples include, but are not limited to, -CH₂-CH₂-O-CH₃, - CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, O-CH₃, -O-CH₂-CH₃, and -CN. Up to two or three heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini (e.g., alkyleneoxo, alkylenedioxo, alkyleneamino, alkylenediamino, and the like). Still further, for alkylene and heteroalkylene linking groups, no orientation of the linking group is implied by the direction in which the formula of the linking group is written. For example, the formula - C(O)OR'- includes both -C(O)OR'- and -R'OC(O)-. As described above, heteroalkyl groups, as used herein, include those groups that are attached to the remainder of the molecule through a heteroatom, such as -C(O)R', -C(O)NR', -NR'R', -OR', -SR', and/or -SO₂R'. Where "heteroalkyl" is recited, followed by recitations of specific heteroalkyl groups, such as -NR'R" or the like, it will be understood that the terms heteroalkyl and -NR'R" are not redundant or mutually exclusive. Rather, the specific heteroalkyl groups are recited to add clarity. Thus, the term "heteroalkyl" should not be interpreted herein as excluding specific heteroalkyl groups, such as -NR'R" or the like.

The terms "cycloalkyl" and "heterocycloalkyl", by themselves or in combination with other terms, re, unless otherwise stated, cyclic versions of "alkyl" and "heteroalkyl", respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like. The terms "cycloalkylene" and "heterocycloalkylene" refer to the divalent derivatives of cycloalkyl and heterocycloalkyl, respectively.

More specifically, the term "alkyl" refers to a branched or unbranched saturated hydrocarbon group of 1 to 6 carbon atoms, such as methyl, ethyl, n-propyl, isopropyl, n-butyl, isobutyl, t-butyl, pentyl, and the like. Alkyl groups herein contain 1 to 6 carbon atoms, such as, for example, methyl, ethyl, and the like. As used herein the term "alkyl" also includes the term "cycloalkyl," which refers to a cyclic alkyl group of three to eight, including three, five or six, carbon atoms. The term "cycloalkylene" as used herein refers to a divalent cyclic alkylene group, typically a 3-, 5-, 6-, or 8-membered ring.

The term "alkoxy" as used herein refers to an alkyl group bound through a single, terminal ether linkage, i.e., an "alkoxy" group may be defined as -OR, where R is alkyl as defined herein. A "lower alkoxy" group refers to an alkoxy group containing 1 to 6, carbon atoms.

The term "aryl" means, unless otherwise stated, a polyunsaturated, aromatic, hydrocarbon substituent which can be a single ring or multiple rings (from 1 to 3 rings) which are fused together or linked covalently. The term "heteroaryl" refers to aryl groups (or rings) that contain from one to four heteroatoms (in each separate ring in the case of multiple rings) selected from N, O, and S, wherein the nitrogen and sulfur atoms are optionally oxidized, and the nitrogen atom(s) are optionally quaternized. A heteroaryl group can be attached to the remainder of the molecule through a carbon or heteroatom. Non-limiting examples of aryl and heteroaryl groups include phenyl, 1-naphthyl, 2-naphthyl, 4-biphenyl, 1-pynolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, and 6-quinolyl. Substituents for each of above noted aryl and heteroaryl ring systems are selected from the group of acceptable substituents described below. The terms "arylene" and "heteroarylene" refer to the divalent radicals of aryl and heteroaryl, respectively.

For brevity, the term "aryl" when used in combination with other terms (e.g., aryloxo, arylthioxo, arylalkyl) includes both aryl and heteroaryl rings as defined above. Thus, the term "arylalkyl" is meant to include those radicals in which an aryl group is attached to an alkyl group (e.g., benzyl, phenethyl, pyridylmethyl and the like) including those alkyl groups in which a carbon atom (e.g., a methylene group) has been replaced by, for example, an oxygen atom (e.g., phenoxymethyl, 2-pyridyloxymethyl, 3-(1-naphthyloxy)propyl, and the like). However, the term "haloaryl," as used herein is meant to cover aryls substituted with one or more halogens.

The term "aryl" as used herein refers to an aromatic carbocyclic ring, typically 6-or 10-membered, wherein at least one ring is aromatic. For example, "aryl" denotes a phenyl group or an ortho-fused bicyclic carbocyclic group having about nine to ten ring atoms in which at least one ring is aromatic.

"Heteroaryl" encompasses a group attached via a ring carbon of a monocyclic aromatic ring containing five or six ring atoms consisting of carbon and one to four heteroatoms each independently may be non-peroxide oxygen, sulfur, and N(X), where X is absent or is H, O, (C₁-C₄)alkyl, phenyl or benzyl, as well as a group of an ortho-fused bicyclic-heterocycle of about eight to ten ring atoms derived therefrom, particularly a benzderivative or one derived by fusing a propylene, trimethylene, or tetramethylene digroup thereto.

Where a heteroalkyl, heterocycloalkyl, or heteroaryl includes a specific number of members (e.g. "3 to 7 membered"), the term "member" referrers to a carbon or heteroatom.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like. The term "halo" also refers to fluoro, chloro, bromo, or iodo.

The term "oxo" as used herein means an oxygen that is double bonded to a carbon atom.

Each of above terms (e.g., "alkyl," "heteroalkyl," "cycloalkyl, and "heterocycloalkyl", "aryl," "heteroaryl" as well as their divalent radical derivatives) are meant to include both substituted and unsubstituted forms of the indicated radical. Substituents for each type of radical are provided below.

Substituents for alkyl, heteroalkyl, cycloalkyl, heterocycloalkyl monovalent and divalent derivative radicals (including those groups often referred to as alkylene, alkenyl, heteroalkylene, heteroalkenyl, alkynyl, cycloalkyl, heterocycloalkyl, cycloalkenyl, and heterocycloalkenyl) can be one or more of a variety of groups selected from, but not limited to: -OR', =O, =NR', =N-OR', -NR'R", -SR', -halogen, -SiR'R"R"', -OC(O)R', -C(O)R', - CO₂R', -C(O)NR'R", -OC(O)NR'R", -NR"C(O)R', -NR'-C(O)NR"R"', - NR"C(O)OR', -NR-C(NR'R")=NR"', -S(O)R', -S(O)₂R' -S(O)₂NR'R" -NRSO₂R', -CN and -NO₂ in a number ranging from zero to (2 m'+1), where m' is the total number of carbon atoms in such radical. R', R", R"' and R"" each independently refer to hydrogen, substituted or unsubstituted heteroalkyl, substituted or unsubstituted cycloalkyl, substituted or unsubstituted heterocycloalkyl, substituted or unsubstituted aryl (e.g., aryl substituted with 1-3 halogens), substituted or unsubstituted alkyl, alkoxy or thioalkoxy groups, or arylalkyl groups. When a compound of the disclosure includes more than one R group, for example, each of the R groups is independently selected as are each R', R", R'" and R"" groups when more than one of these groups is. When R' and R"' are attached to the same nitrogen atom, they can be combined with the nitrogen atom to form a 4-, 5-, 6-, or 7-membered ring. For example, -NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl (e.g., -CF₃ and -CH₂CF₃) and acyl (e.g., - C(O)CH-, -C(O)CF₃, -C(O)CH₂OCH₃, and the like).

Similar to the substituents described for alkyl radicals above, exemplary substituents for aryl and heteroaryl groups (as well as their divalent derivatives) are varied and are selected from, for example: halogen, -OR', -NR'R", -SR', -halogen, -SiR'R"R"', - OC(O)R', -C(O)R', -CO₂R' -C(O)NR'R", -OC(O)NR'R", -NR"C(O)R', -NR'-C(O)NR"R"', -NR"C(O)OR', -NR-C(NR'R"R"')=NR"", -NR-C(NR'R") =NR"', -S(O)R', -S(O)₂R', -S(O)₂NR'R", -NRSO₂R' -CN and -NO₂, -R', -N₃, -CH(Ph)₂, fluoro(C₁-C₄)alkoxy, and fluoro(C₁-C₄)alkyl, in a number ranging from zero to the total number of open valences on aromatic ring system; and where R', R", R'" and R"" are independently selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl and substituted or unsubstituted heteroaryl. When a compound of the disclosure includes more than one R' group, for example, each of the R groups is independently selected as are each R', R", R'" and R"" groups when more than one of these groups is.

Two of the substituents on adjacent atoms of aryl or heteroaryl ring may optionally form a ring of the formula -T-C(O)-(CRR')_{q}-U-, wherein T and U are independently -NR-, -O-, -CRR'- or a single bond, and q is an integer of from 0 to 3. Alternatively, two of the substituents on adjacent atoms of aryl or heteroaryl ring may optionally be replaced with a substituent of the formula -A-(CH₂), -B-, wherein A and B are independently -CRR'-, -O-, -NR-, -S-, -S(O)-, -S(O)₂, -S(O)₂NR'- or a single bond, and r is an integer of from 1 to 4. One of the single bonds of the new ring so formed may optionally be replaced with a double bond. Alternatively, two of the substituents on adjacent atoms of aryl or heteroaryl ring may optionally be replaced with a substituent of the formula -(CRR')₅-X'-(C"R"')_{d}-, where s and d are independently integers of from 0 to 3, and X' is -O-, -NR'-, -S-, -S(O)-, -S(O)₂-, or -S(O)₂NR'-. The substituents R, R', R" and R"' are independently selected from hydrogen, substituted or unsubstituted alkyl, substituted or unsubstituted cycloalkyl, substituted or unsubstituted heterocycloalkyl, substituted or unsubstituted aryl, and substituted or unsubstituted heteroaryl.

As used herein, the term "heteroatom" or "ring heteroatom" is meant to include oxygen (O), nitrogen (N), sulfur (S), phosphorus (P), and silicon (Si).

An "aminoalkyl" as used herein refers to an amino group covalently bound to an alkylene linker. The amino group is -NR'R", wherein R' and R" are typically selected from hydrogen, substituted or unsubstituted alkyl, substituted or unsubstituted heteroalkyl, substituted or unsubstituted cycloalkyl, substituted or unsubstituted heterocycloalkyl, substituted or unsubstituted aryl, or substituted or unsubstituted heteroaryl.

A "substituent group," as used herein, means a group selected from the following moieties:
(A) -OH, -NH₂, -SH, -CN, -CF₃, -NO₂, oxo, halogen, unsubstituted alkyl, unsubstituted heteroalkyl, unsubstituted cycloalkyl, unsubstituted heterocycloalkyl, unsubstituted aryl, unsubstituted heteroaryl, and
(B) alkyl, heteroalkyl, cycloalkyl, heterocycloalkyl, aryl, and heteroaryl, substituted with at least one substituent selected from:
   (i) oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and
   (ii) alkyl, heteroalkyl, cycloalkyl, heterocycloalkyl, aryl, and heteroaryl, substituted with at least one substituent selected from:
      (a) oxo, -OH, -NH₂, -SH, -CN, - CF₃, -NO₂, halogen, unsubstituted alkyl, unsubstituted heteroalkyl, unsubstituted cycloalkyl, unsubstituted heterocycloalkyl, unsubstituted aryl, unsubstituted heteroaryl, and
      (b) alkyl, heteroalkyl, cycloalkyl, heterocycloalkyl, aryl, or heteroaryl, substituted with at least one substituent selected from oxo, -OH, -NH₂, -SH, -CN, -CF₃, -NO₂, halogen, unsubstituted alkyl, unsubstituted heteroalkyl, unsubstituted cycloalkyl, unsubstituted heterocycloalkyl, unsubstituted aryl, and unsubstituted heteroaryl.

A "size-limited substituent" or "size-limited substituent group," as used herein means a group selected from all of the substituents described above for a "substituent group," wherein each substituted or unsubstituted alkyl is a substituted or unsubstituted C₁-C₂₀ alkyl, each substituted or unsubstituted heteroalkyl is a substituted or unsubstituted 2 to 20 membered heteroalkyl, each substituted or unsubstituted cycloalkyl is a substituted or unsubstituted C₄-C₈ cycloalkyl, and each substituted or unsubstituted heterocycloalkyl is a substituted or unsubstituted 4 to 8 membered heterocycloalkyl.

A "lower substituent" or "lower substituent group," as used herein means a group selected from all of the substituents described above for a "substituent group," wherein each substituted or unsubstituted alkyl is a substituted or unsubstituted C₁-C₈ alkyl, each substituted or unsubstituted heteroalkyl is a substituted or unsubstituted 2 to 8 membered heteroalkyl, each substituted or unsubstituted cycloalkyl is a substituted or unsubstituted C₅-C₇ cycloalkyl, and each substituted or unsubstituted heterocycloalkyl is a substituted or unsubstituted 5 to 7 membered heterocycloalkyl.

The compounds of the disclosure may exist as salts. The disclosure includes such salts. Examples of applicable salt forms include hydrochlorides, hydrobromides, sulfates, methanesulfonates, nitrates, maleates, acetates, citrates, fumarates, tartrates (eg (+)-tartrates, (-)-tartrates or mixtures thereof including racemic mixtures, succinates, benzoates and salts with amino acids such as glutamic acid. These salts may be prepared by methods known to those skilled in art. Also included are base addition salts such as sodium, potassium, calcium, ammonium, organic amino, or magnesium salt, or a similar salt. When compounds of the disclosure contain relatively basic functionalities, acid addition salts can be obtained by contacting the neutral form of such compounds with a sufficient amount of the desired acid, either neat or in a suitable inert solvent. Examples of acceptable acid addition salts include those derived from inorganic acids like hydrochloric, hydrobromic, nitric, carbonic, monohydrogen-carbonic, phosphoric, monohydrogenphosphoric, dihydrogenphosphoric, sulfuric, mono-hydrogensulfuric, hydriodic, or phosphorous acids and the like, as well as the salts derived organic acids like acetic, propionic, isobutyric, maleic, malonic, benzoic, succinic, suberic, fumaric, lactic, mandelic, phthalic, benzenesulfonic, p-tolylsulfonic, citric, tartaric, methane-sulfonic, and the like. Also included are salts of amino acids such as arginate and the like, and salts of organic acids like glucuronic or galactunoric acids and the like. Certain specific compounds of the disclosure contain both basic and acidic functionalities that allow the compounds to be converted into either base or acid addition salts.

The neutral forms of the compounds are regenerated by contacting the salt with a base or acid and isolating the parent compound in the conventional manner. The parent form of the compound differs from the various salt forms in certain physical properties, such as solubility in polar solvents.

Certain compounds of the disclosure can exist in unsolvated forms as well as solvated forms, including hydrated forms. In general, the solvated forms are equivalent to unsolvated forms and are encompassed within the scope of the disclosure. Certain compounds of the disclosure may exist in multiple crystalline or amorphous forms. In general, all physical forms are equivalent for the uses contemplated by the disclosure and are intended to be within the scope of the disclosure.

Certain compounds of the disclosure possess asymmetric carbon atoms (optical or chiral centers) or double bonds; the enantiomers, racemates, diastereomers, tautomers, geometric isomers, stereoisometric forms that may be defined, in terms of absolute stereochemistry, as (R)- or (S)- or, as (D)- or (L)- for amino acids, and individual isomers are encompassed within the scope of the disclosure. The compounds of the disclosure do not include those which are known in art to be too unstable to synthesize and/or isolate. The disclosure is meant to include compounds in racemic and optically pure forms. Optically active (R)- and (S)-, or (D)- and (L)-somers may be prepared using chiral synthons or chiral reagents, or resolved using conventional techniques. When the compounds described herein contain olefinic bonds or other centers of geometric asymmetry, and unless specified otherwise, it is intended that the compounds include both E and Z geometric isomers.

The term "tautomer," as used herein, refers to one of two or more structural isomers which exist in equilibrium and which are readily converted from one isomeric form to another.

It will be apparent to one skilled in the art that certain compounds of this disclosure may exist in tautomeric forms, all such tautomeric forms of the compounds being within the scope of the disclosure.

Unless otherwise stated, structures depicted herein are also meant to include all stereochemical forms of the structure; i.e., the R and S configurations for each asymmetric center. Therefore, single stereochemical isomers as well as enantiomeric and diastereomeric mixtures of the compounds are within the scope of the disclosure.

Unless otherwise stated, structures depicted herein are also meant to include compounds which differ in the presence of one or more isotopically enriched atoms. For example, compounds having the structures except for the replacement of a hydrogen by a deuterium or tritium, or the replacement of a carbon by ¹³C- or ^{l14}C-enriched carbon are within the scope of this disclosure.

The compounds of the disclosure may also contain unnatural proportions of atomic isotopes at one or more of atoms that constitute such compounds. For example, the compounds may be radiolabeled with radioactive isotopes, such as for example tritium (3H), iodine-125 (¹²⁵I) or carbon-14 (¹⁴C). All isotopic variations of the compounds of the disclosure, whether radioactive or not, are encompassed within the scope of the disclosure.

Throughout the present disclosure the term "about" a certain value means that a range of value±25%, and preferably a range of value±10%, and more preferably a range of value±5%, is contemplated. Thus, for example, about 20 mol % of a certain reagent includes the reagent being present between 15% and 25%, preferably between 18% and 22%, and more preferably between 19% and 73.5%.

The terms "a," "an," or "a(n)", when used in reference to a group of substituents herein, mean at least one. For example, where a compound is substituted with "an" alkyl or aryl, the compound is optionally substituted with at least one alkyl and/or at least one aryl. Moreover, where a moiety is substituted with an R substituent, the group may be referred to as "R-substituted." Where a moiety is R-substituted, the moiety is substituted with at least one R substituent and each R substituent is optionally different.

Description of compounds of the disclosure are limited by principles of chemical bonding known to those skilled in the art. Accordingly, where a group may be substituted by one or more of a number of substituents, such substitutions are selected so as to comply with principles of chemical bonding and to give compounds which are not inherently unstable and/or would be known to one of ordinary skill in the art as likely to be unstable under ambient conditions, such as aqueous, neutral, and several known physiological conditions. For example, a heterocycloalkyl or heteroaryl is attached to the remainder of the molecule via a ring heteroatom in compliance with principles of chemical bonding known to those skilled in the art thereby avoiding inherently unstable compounds.

It will be appreciated by those skilled in the art that compounds of the disclosure having a chiral center may exist in and be isolated in optically active and racemic forms. Some compounds may exhibit polymorphism. It is to be understood that the disclosure encompasses any racemic, optically active, polymorphic, or stereoisomeric form, or mixtures thereof, of a compound of the disclosure, which possesses the useful properties described herein. Also, if the named compound comprises a chiral center, the scope of the disclosure also includes compositions comprising the racemic mixture of the two enantiomers, as well as compositions comprising each enantiomer individually, substantially free of the other enantiomer. Thus, for example, contemplated herein is a composition comprising the S enantiomer substantially free of the R enantiomer, or a composition comprising the R enantiomer substantially free of the S enantiomer.

By "substantially free" it is meant that the composition comprises less than 10%, or less than 8%, or less than 5%, or less than 3%, or less than 1% of the minor enantiomer. If the named compound comprises more than one chiral center, the scope of the disclosure also includes compositions comprising a mixture of the various diastereomers, as well as compositions comprising each diastereomer substantially free of the other diastereomers.

It is well known in the art how to prepare optically active forms (for example, by resolution of the racemic form by recrystallization techniques, by synthesis from optically active starting materials, by chiral synthesis, or by chromatographic separation using a chiral stationary phase) and how to determine the anti cancer activity using the standard tests described herein, or using other similar tests which are well known in the art.

As used herein, "substantially pure" means an object species is the predominant species (i.e., on a molar basis it is more abundant than any other individual species in the composition), and a substantially purified fraction is a composition wherein the object species comprises at least about 50 percent (on a molar basis) of all macromolecular species. Generally, a substantially pure composition will comprise more than about 80 percent of all macromolecular species in the composition, for example, more than about 85%, 90%, 95%, and 99%. The object species may be also purified to essential homogeneity (contaminant species cannot be detected in the composition by conventional detection methods), wherein the composition consists essentially of a single species.

Where the antimicrobial peptides are to be used as antimicrobial agents, they can be formulated, for example, in buffered aqueous media containing a variety of salts and buffers. Examples of the salts include, but are not limited to, halides, phosphates and sulfates, e.g., sodium chloride, potassium chloride or sodium sulfate. Various buffers may be used, such as citrate, phosphate, HEPES, Tris or the like to the extent that such buffers are physiologically acceptable to the host that is being treated.

Various excipients or other additives may be used, where the peptides are formulated as lyophilized powders, for subsequent use in solution. The excipients may include various polyols, inert powders or other extenders.

"Therapeutically effective" as used herein, refers to an amount of formulation, composition, or reagent, optionally in a pharmaceutically acceptable carrier, that is of sufficient quantity to ameliorate the state of the patient or animal so treated. "Ameliorate" refers to a lessening of the detrimental effect of the disease state or disorder in the recipient of the therapy. In an embodiment, a peptide of the invention is administered to a subject in need of treatment.

Pharmaceutically acceptable carrier preparations for administration include sterile or aqueous or nonaqueous solutions, suspensions, and emulsions. Examples of nonaqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solutions, Ringer's dextrose, dextrose and sodium chloride, lactated Ringer's, or fixed oils. Active therapeutic ingredients are often mixed with excipients that are pharmaceutically acceptable and compatible with the active ingredients. Suitable excipients include water, saline, dextrose, glycerol and ethanol, or combinations thereof. Intravenous vehicles include fluid and nutrient replenishers, electrolyte replenishers, such as those based on Ringer's dextrose, and the like. Preservatives and other additives may also be present such as, for example, antioxidants, chelating agents, and inert gases and the like. The actual dosage of the peptides, formulations or compositions containing such peptides can depend on many factors, including the size/weight, age, and health of an organism, however, one of ordinary skill in the art can use the following teachings and others known in the art describing the methods and techniques for determining clinical dosages (Spiker B., Guide to Clinical Studies and Developing Protocols, Raven Press, Ltd., New York, 1984, pp. 7-13, 54-60; Spiker B., Guide to Clinical Trials, Raven Press, Ltd., New York 1991, pp. 93-101; C. Craig. and R. Stitzel, eds., Modern Pharmacology, 2d ed., Little, Brown and Co., Boston, 1986, pp. 127-133; T. Speight, ed., Avery's Drug Treatment: Principles and Practice of Clinical Pharmacology and Therapeutics, 3d ed., Williams and Wilkins, Baltimore, 1987, pp. 50-56; R. Tallarida, R. Raffa and P. McGonigle, Principles in General Pharmacology, Springer-Verlag, new York, 1988, pp. 18-20) to determine the appropriate dosage to use.

As used herein, the term "amino acid" is intended to refer to any natural or unnatural amino acid, whether made naturally or synthetically, including any such in L- or D-configuration. The term can also encompass amino acid analog compounds used in peptidomimetics or in peptoids. The term can include a modified or unusual amino acid or a synthetic derivative of an amino acid, e.g. diamino butyric acid and diamino propionic acid and the like.

The disclosed antimicrobial peptides are composed of amino acids linked together by peptide bonds. Sequences are conventionally given from the amino terminus to the carboxyl terminus. Unless otherwise noted, the amino acids are L-amino acids. When all the amino acids are of L-configuration, the peptide is said to be an L-enantiomer. When all the amino acids are of D-configuration, the peptide is said to be a D-enantiomer.

The term "minimal inhibitory concentration" (MIC) refers to the lowest concentration of an antimicrobial agent (e.g., a peptide) required to prevent growth or otherwise modify a function of a microorganism under certain conditions, for example in liquid broth medium, and can be determined for a number of different microorganisms according to standard techniques well known in the art.

The term "minimal hemolytic concentration" (MHC) refers to the lowest concentration of an agent or peptide required to cause hemolysis of blood cells. MHC can be determined with red blood cells (RBC) from various species including human red blood cells (hRBC).

The term "therapeutic index" (TI) is the ratio of minimal hemolytic concentration (MHC) over minimal inhibitory concentration (MIC) of an antimicrobial agent. Larger values generally indicate greater antimicrobial specificity.

The term "stability" can refer to an ability to resist degradation, to persist in a given environment, and/or to maintain a particular structure. For example, a peptide property of stability can indicate resistance to proteolytic degradation and to maintain an alpha-helical structural conformation.

The following abbreviations are used herein: A, Ala, Alanine; M, Met, Methionine; C, Cys, Cysteine; D, Asp, Aspartic Acid; E, Glu, Glutamic Acid; F, Phe, Phenylalanine; G, Gly, Glycine; H, His, Histidine; I, Ile, Isoleucine; K, Lys, Lysine; L, Leu, Leucine; N, Asn, Asparagine; P, Pro, Proline; Q, Glu, Glutamine; R, Arg, Arginine; S, Ser, Serine; T, Thr, Threonine; V, Val, Valine; W, Trp, Tryptophan; Y, Tyr, Tyrosine; RP-HPLC, reversed-phase high performance liquid chromatography; MIC, minimal inhibitory concentration; MHC, minimal hemolytic concentration; CD, circular dichroism spectroscopy; TFE, trifluoroethanol; TFA, trifluoroacetic acid; RBC, red blood cells; hRBC, human red blood cells.

The term "antimicrobial activity" refers to the ability of a peptide of the present invention to modify a function or metabolic process of a target microorganism, for example so as to at least partially affect replication, vegetative growth, toxin production, survival, viability in a quiescent state, or other attribute. In an embodiment, the term relates to inhibition of growth of a microorganism. In a particular embodiment, antimicrobial activity relates to the ability of an inventive peptide to kill at least one bacterial species. In a particular embodiment, the bacterial species is selected from the group consisting of gram-positive and gram-negative bacteria. In an embodiment, the term can be manifested as microbicidal or microbistatic inhibition of microbial growth.

The phrase "improved biological property" is meant to indicate that a test peptide exhibits less hemolytic activity and/or better antimicrobial activity, or better antimicrobial activity and/or less hemolytic activity, compared to the control peptide (e.g. V.sub.681), when tested by the protocols described herein or by any other art-known standard protocols. In general, the improved biological property of the peptide is reflected in the therapeutic index (TI) value which is better that that of the control peptide.

The term "microorganism" herein refers broadly to bacteria, fungi, viruses, and protozoa. In particular, the term is applicable for a microorganism having a cellular or structural component of a lipid bilayer membrane. In specific embodiments, the membrane is a cytoplasmic membrane. Pathogenic bacteria, fungi, viruses, and protozoa as known in the art are generally encompassed. Bacteria can include gram-negative and gram-positive bacteria in addition to organisms classified in orders of the class Mollicutes and the like, such as species of the Mycoplasma and Acholeplasma genera. Specific examples of potentially sensitive gram-negative bacteria include, but are not limited to, Escherichia coli, Pseudomonas aeruginosa, Salmonella, Hemophilus influenza, Neisseria, Vibrio cholerae, Vibrio parahaemolyticus and Helicobacter pylori. Examples of potentially sensitive gram-positive bacteria include, but are not limited to, Staphylococcus aureus, Staphylococcus epidermis, Streptococcus agalactiae, Group A streptococcus, Streptococcus pyogenes, Enterococcus faecalis, Group B gram positive streptococcus, Corynebacterium xerosis, and Listeria monocytogenes. Examples of potentially sensitive fungi include yeasts such as Candida albicans. Examples of potentially sensitive viruses include measles virus, herpes simplex virus (HSV-1 and -2), herpes family members (HIV, hepatitis C, vesicular, stomatitis virus (VSV), visna virus, and cytomegalovirus (CMV). Examples of potentially sensitive protozoa include Giardia.

"Therapeutically effective" as used herein, refers to an amount of formulation, composition, or reagent in a pharmaceutically acceptable carrier or a physiologically acceptable salt of an active compound, that is of sufficient quantity to ameliorate the undesirable state of the patient, animal, material, or object so treated. "Ameliorate" refers to a lessening of the detrimental effect of the disease state or disorder, or reduction in contamination, in the receiver of the treatment.

The antimicrobial peptides of the invention have antimicrobial activity by themselves or when covalently conjugated or otherwise coupled or associated with another molecule, e.g., polyethylene glycol or a carrier protein such as bovine serum albumin, so long as the peptides are positioned such that they can come into contact with a cell or unit of the target microorganism. These peptides may be modified by methods known in the art provided that the antimicrobial activity is not destroyed or substantially compromised.

Chitosan is a linear polysaccharide composed of randomly distributed β-(1-4)-linked D-glucosamine (deacetylated unit) and N-acetyl-D-glucosamine (acetylated unit). Chitosan is produced commercially by deacetylation of chitin, which is the structural element in the exoskeleton of crustaceans such as crabs and shrimp, and cell walls of fungi. The degree of deacetylation (%DD) may be determined by NMR spectroscopy. The %DD in commercial chitosans ranges from 60 to 100%. On average, the molecular weight of commercially produced chitosan is between 3,800 and 20,000 Daltons. A common method for the synthesis of chitosan is the deacetylation of chitin using sodium hydroxide in excess as a reagent and water as a solvent. This reaction pathway, when allowed to go to completion (complete deacetylation) yields up to 98% product

Bacteria are traditionally divided into two main groups: Gram-positive and Gram-negative bacteria, based on their Gram-stain retention properties. The test is useful in classifying these two distinct types of bacteria based on the structural differences of their cell walls. Gram-positive bacteria retain the crystal violet dye in the Gram staining protocol and are stained dark blue or violet due to the high amount of peptidoglycan in their cell wall. By contrast, Gram-negative bacteria do not retain the crystal violet stain but instead, take up the counterstain (safranin or fuchsine) and appear red or pink due to their outer membrane of lipopolysaccharide and protein that surrounds the peptidoglycan layer. Although bacteria are traditionally divided into these two main groups, this classification system is ambiguous as it can refer to three distinct aspects (staining result, cell-envelope organization, taxonomic group), which do not necessarily coalesce for some bacterial species. The Gram-positive and Gram-negative staining response is also not a reliable characteristic as these two kinds of bacteria do not form phylogenetic coherent groups.

While Gram-staining response of bacteria is an empirical criterion, its basis lies in the marked differences in the ultrastructure and chemical composition of the two main kinds of prokaryotic cells that are found in nature. These two kinds of cells are distinguished from each other based upon the presence or absence of an outer lipid membrane, which is a more reliable and fundamental characteristic of the bacterial cells. Gram-positive bacteria are bounded by only a single unit lipid membrane and they generally contain a thick layer (20-80 nm) of peptidoglycan responsible for retaining the Gram-stain. A number of other bacteria which are bounded by a single membrane, but which stain Gram-negative due to either lack of the peptidoglycan layer (mycoplasmas) or their inability to retain the Gram-stain due to their cell wall composition, also show close relationship to the gram-positive bacteria.

Most pathogens in humans are Gram-positive organisms. Six Gram-positive bacteria are typically pathogenic in humans. Two of these, *Streptococcus* and *Staphylococus,* are cocci (sphere-shaped bacteria). The remaining bacteria are bacilli (rod-shaped bacteria) and can be subdivided based on their ability to form spores. The non-spore formers are *Corynebacterium* and *Listeria* (a coccobacillus), whereas *Bacillus* and *Clostridum* produce spores. The spore-forming bacteria can again be divided based on their respiration: *Bacillus* is a facultative anaerobe, while *Clostridium* is an anobligate anaerobe.

In contrast to Gram-positive bacteria, Gram-negative bacteria are bounded by both a cytoplasmic membrane as well as an outer cell membrane and they contain only a thin layer of peptidoglycan (2-3 nm) in between these two membranes. The presence of both inner and outer cell membranes defines a new compartment in these cells, the periplasmic space or compartment.

The proteobacteria are a major group of Gram-negative bacteria, which includes *Escherichia coli* (E. coli), *Salmonella, Shigella,* and other Enterobacteriaceae, *Pseudomonas, Moraxella, Helicobacter,Stenotrophomonas, Bdellovibrio,* acetic acid bacteria, *Legionella* and numerous others. Other notable groups of Gram-negative bacteria include the cyanobacteria, spirochaetes, green sulfur and green non-sulfur bacteria. Medically relevant Gram-negative cocci include three organisms, which cause a sexually transmitted disease *(Neisseria gonorrhoeae),* a meningitis *(Neisseria meningitidis),* and respiratory symptoms *(Moraxella catarrhalis).* Medically relevant Gram-negative bacilli include a multitude of species. Some of them primarily cause respiratory problems *(Hemophilus influenzae, Klebsiella pneumoniae, Legionella pneumophila, Pseudomonas aeruginosa*), primarily urinary problems *(Escherichia coli, Proteus mirabilis, Enterobacter cloacae, Serratia marcescens),* and primarily gastrointestinal problems *(Helicobacter pylori, Salmonella enteritidis, Salmonella typhi).* Gram-negative bacteria associated with nosocomial infections include *Acinetobacter baumannii,* which cause bacteremia, secondary meningitis, and ventilator-associated pneumonia in intensive-care units of hospital establishments.

### Antimicrobial Peptides (AMPs)

Most AMPs are characterized by their cationic charge and their amphiphilic nature. These two characteristics allow AMPs to be electrostatically attracted to the anionic cytoplasmic membrane and then, penetrate into the hydrophobic lipid bilayer interior of a microbe. Numerous chemical structures have been designed and synthesized to mimic the cationic and amphiphilic nature of natural AMPs; these include synthetic peptides, peptoids, polymethacrylates, polypryidines, polynorbornenes, polysaccharides, and the like. Many synthetic efforts have also considered the electrostatic interaction of an AMP with the cytoplasmic membrane of a microbe but have largely ignored their compatibility with the complex cell wall structure. In addition, the relatively low selectivity of most synthetic AMPs results in cytotoxicity to mammalian cells (more specifically, by hemolysis of red blood cells).

Peptidoglycans are a common component of the bacteria cell wall, a feature absent from animal cell walls. For example, both Gram-negative and Gram-positive bacteria have a peptidoglycan layer outside the cytoplasmic membrane. AMPs that are designed to resemble bacterial cell wall constituents may be less hemolytic than the present AMPs due to their dissimilarity to mammalian outer membrane constituents. To date, peptidopolysaccharides that have a peptidoglycan-mimetic chemical structure for facilitating penetration into the bacterial cell wall have not been used as antimicrobial biomacromolecules.

It has been surprisingly found, however, that compounds or polymers that mimic the peptidoglycan structure may be useful as antimicrobial agents. Accordingly, the disclosure provides an unexpected new class of antimicrobial polymer type compounds based on a cationic peptidopolysaccharide, which mimics the peptidoglycan structure of bacterial cell walls, and which show excellent antimicrobial activity and selectivity as well as low hemolytic activity.

Thus, in one embodiment the disclosure provides compounds having Formula I: 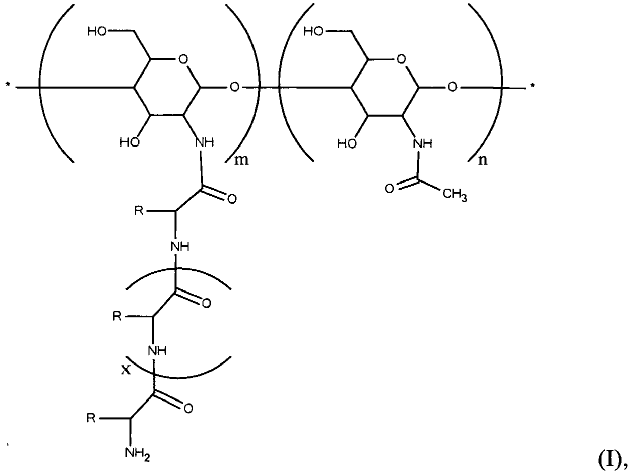 or a pharmaceutically acceptable salt thereof, wherein:
each R is independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, (CH₂)ⱼOR¹, (CH₂)ⱼC(O)R¹, (CH₂)ⱼC(O)OR¹, (CH₂)ⱼOC(O)R¹, (CH₂)ⱼNR²R³, (CH₂)ⱼC(O)NR²R³, (CH₂)ⱼOC(O)NR²R³, (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ and (CH₂)ⱼNR⁴S(O)ₖNR²R³, wherein j is an integer independently selected from 0 to 6, k is an integer independently selected from 0 to 2, and wherein each R group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
R¹, R², R³ and R⁴ are each independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, wherein each R¹, R², R³ and R⁴ group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
m is an integer independently selected from 1 to 100,000;
n is an integer independently selected from 1 to 100,000; and
x is an integer independently selected from 1 to 5,000.

In another embodiment the disclosure provides compounds having Formula I, wherein m and n are each an integer, each independently selected from 1 to 90,000, or m and n are each an integer independently selected from 1 to 80,000, or m and n are each an integer independently selected from 1 to 70,000, or m and n are each an integer independently selected from 1 to 60,000, or m and n are each an integer independently selected from 1 to 90,000, or m and n are each an integer independently selected from 1 to 50,000, or m and n are each an integer independently selected from 1 to 40,000, or m and n are each an integer independently selected from 1 to 30,000, or m and n are each an integer independently selected from 1 to 20,000, or m and n are each an integer independently selected from 1 to 10,000, or m and n are each an integer independently selected from 1 to 5,000, or m and n are each an integer independently selected from 1 to 3,000, or m and n are each an integer independently selected from 1 to 2,000, or m and n are each an integer independently selected from 1 to 1,000, or m and n are each an integer independently selected from 1 to 500, or m and n are each an integer independently selected from 1 to 250, or m and n are each an integer independently selected from 1 to 100, or m and n are each an integer independently selected from 1 to 50, or m and n are each an integer independently selected from 1 to 25, or m and n are each an integer independently selected from 1 to 20, or m and n are each an integer independently selected from 1 to 10, or m and n are each an integer independently selected from 1 to 5, or m and n are each an integer independently selected from 1, 2, 3, 4 and 4; and

x is an integer independently selected from 1 to 4,000, or x is an integer independently selected from 1 to 3,000, or x is an integer independently selected from 1 to 2,500, or x is an integer independently selected from 1 to 2,000, or x is an integer independently selected from 1 to 1,500, or x is an integer independently selected from 1 to 1,000, or x is an integer independently selected from 1 to 900, or x is an integer independently selected from 1 to 800, or x is an integer independently selected from 1 to 750, or x is an integer independently selected from 1 to 700, or x is an integer independently selected from 1 to 600, or x is an integer independently selected from 1 to 500, or x is an integer independently selected from 1 to 400, or x is an integer independently selected from 1 to 300, or x is an integer independently selected from 1 to 250, or x is an integer independently selected from 1 to 200, or x is an integer independently selected from 1 to 100, or x is an integer independently selected from 1 to 75, or x is an integer independently selected from 1 to 50, or x is an integer independently selected from 1 to 40, or x is an integer independently selected from 1 to 30, or x is an integer independently selected from 1 to 25, or x is an integer independently selected from 1 to 20, or x is an integer independently selected from 1 to 15, or x is an integer independently selected from 1 to 10, or x is an integer independently selected from 1 to 5, or x is an integer independently selected from 1, 2, 3, 4 and 4.

In another embodiment the disclosure provides compounds having Formula I, wherein:
R is independently selected from substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, substituted or unsubstituted (C₃-C₁₀)heteroaryl(C₁-C₆)alkyl, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ and (CH₂)NR⁴S(O)ₖNR²R³; and
R¹, R², R³ and R⁴ are each independently selected from hydrogen, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, and substituted or unsubstituted (C₃-C₁₀)heteroaryl(C₁-C₆)alkyl.

In another embodiment the disclosure provides compounds having Formula I, wherein:
R is independently selected from (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, hetero(C₃-C₁₀)aryl, hetero(C₃-C₁₀)aryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR⁴S(O)ₖCH₃, S(O)ₖNR²R³ and NR⁴S(O)ₖNR²R³; and
R¹, R², R³ and R⁴ are each independently selected from hydrogen, (C₁-C₆)alkyl, (C₂-C₆)alkenyl, (C₂-C₆)alkynyl, (C₃-C₈)cycloalkyl, (C₆-C₁₀)aryl], (C₆-C₁₀)aryl](C₁-C₆)alkyl, (C₃-C₁₀)heteroaryl, and (C₃-C₁₀)heteroaryl(C₁-C₆)alkyl.

In another embodiment the disclosure provides compounds having Formula I, wherein R is independently selected from CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH and CH₂SCH₃.

In another embodiment the disclosure provides compounds having Formula I, wherein the compound of Formula I is selected from:

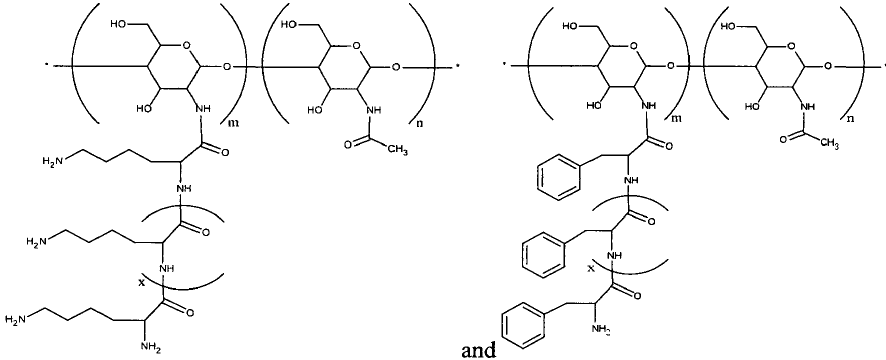

In another embodiment the disclosure provides pharmaceutical compositions including a compound of Formula I, and a pharmaceutically acceptable carrier.

In another embodiment the disclosure provides therapeutic compositions for controlling infection by a microorganism, including the compound of Formula I in a therapeutically effective amount and a pharmaceutically acceptable carrier.

In another embodiment the disclosure provides therapeutic compositions for controlling infection by a microorganism, including the compound of Formula I in a therapeutically effective amount and a pharmaceutically acceptable carrier, wherein the microorganism is selected from a bacterium, a fungus, a virus, and a protozoan.

Also disclosed within the context of the present invention are methods for controlling a microorganism by administering a therapeutically effective amount of a composition, wherein the composition includes the compound of Formula I and an acceptable carrier.

Also disclosed within the context of the present invention are methods for controlling a microorganism by administering a therapeutically effective amount of a composition, wherein the composition includes the compound of Formula I and an acceptable carrier, wherein the microorganism is selected from a gram-negative bacterium and a gram-positive bacterium.

Also disclosed within the context of the present invention are methods for treating a subject in need of or preventing an infection in a subject caused by a microorganism by administering a therapeutically effective amount of a pharmaceutical composition of the compound of Formula I to a subject with the infection.

Also disclosed within the context of the present invention are methods for treating a subject in need of or preventing an infection in a subject caused by a microorganism by administering a therapeutically effective amount of a pharmaceutical composition of the compound of Formula I to a subject with the infection, wherein the microorganism is selected from gram-positive bacteria and gram-negative bacteria.

In another embodiment the disclosure provides ex-vivo methods for disinfecting a surface of an article by applying to the surface an effective amount of a composition, wherein the composition includes the compound of Formula I.

In another embodiment the disclosure provides disinfecting solutions including the compound of Formula I, and optionally an acceptable carrier.

In another embodiment the disclosure provides methods for preparing a compound of Formula I, or a pharmaceutically acceptable salt thereof by:
a) reacting a compound of Formula II with a compound of Formula III to provide a compound of Formula IV; and
b) deprotecting the compound of Formula IV to provide the compound of Formula I: 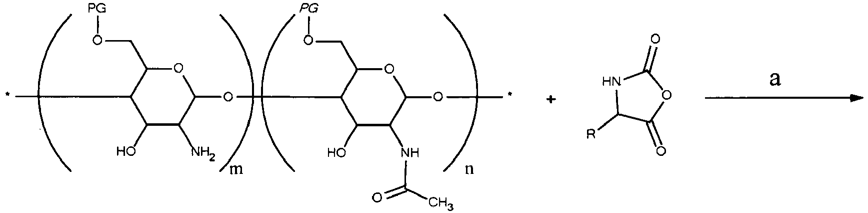 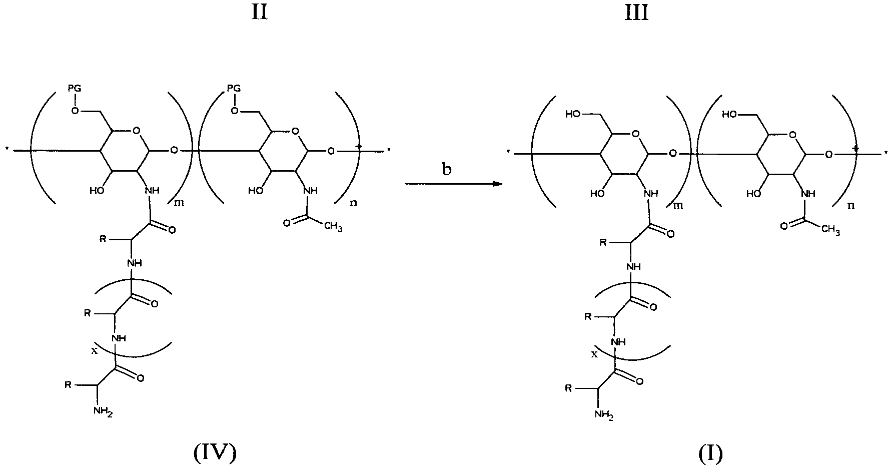 wherein:
   each PG is a protecting group;
   each R is independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, (CH₂)ⱼOR¹, (CH₂)ⱼC(O)R¹, (CH₂)ⱼC(O)OR¹, (CH₂)ⱼOC(O)R¹, (CH₂)ⱼNR²R³, (CH₂)ⱼC(O)NR²R³, (CH₂)ⱼOC(O)NR²R³, (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ and (CH₂)ⱼNR⁴S(O)ₖNR²R³, wherein j is an integer independently selected from 0 to 6, k is an integer independently selected from 0 to 2, and wherein each R group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
   R¹, R², R³ and R⁴ are each independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, wherein each R¹, R², R³ and R⁴ group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
   m is an integer independently selected from 1 to 100,000;
   n is an integer independently selected from 1 to 100,000; and
   x is an integer independently selected from 1 to 5,000.

In another embodiment the disclosure provides methods for preparing a compound of Formula I, wherein:
PG is a triphenylmethyl protecting group;
R is independently selected from substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, substituted or unsubstituted (C₃-C₁₀)heteroaryl(C₁-C₆)alkyl, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ and (CH₂)NR⁴S(O)ₖNR²R³; and
R¹, R², R³ and R⁴ are each independently selected from hydrogen, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, and substituted or unsubstituted (C₃-C₁₀)heteroaryl(C₁-C₆)alkyl.

In another embodiment the disclosure provides methods for preparing a compound of Formula I, wherein:
R is independently selected from (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, hetero(C₃-C₁₀)aryl, hetero(C₃-C₁₀)aryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR⁴S(O)ₖCH₃, S(O)ₖNR²R³ and NR⁴S(O)ₖNR²R³; and
R¹, R², R³ and R⁴ are each independently selected from hydrogen, (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, (C₃-C₁₀)heteroaryl, and (C₃-C₁₀)heteroaryl(C₁-C₆)alkyl.

In another embodiment the disclosure provides methods for preparing a compound of Formula I, wherein R is independently selected from CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH², (CH₂)₃NHC(NH)NH₂, CH₂(C₃H₂N₂), CH₂(C₈H₆N), CH₂SH and CH₂SCH₃.

In another embodiment the disclosure provides methods for preparing a compound of Formula I, wherein the compound of Formula I is selected from:

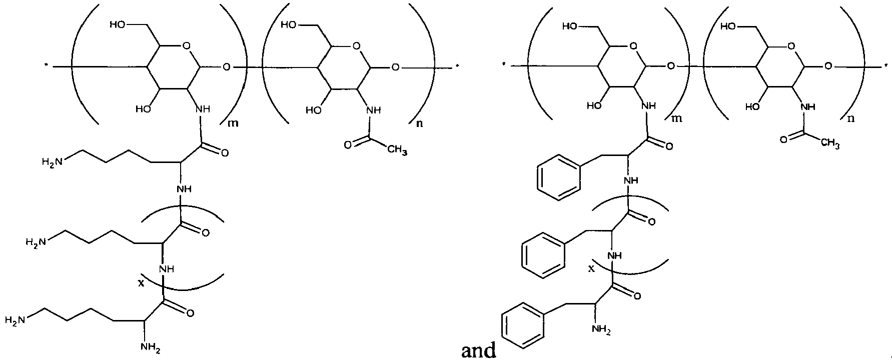

In another embodiment the disclosure provides a compound of Formula I, prepared by the methods disclosed herein.

Any of the following dosages may be used in the disclosed methods: generally in the range of about 0.001 mg/kg to about 100 mg/kg; or from about 0.001 mg/kg to about 1 mg/kg final concentration are administered per day to an adult in any pharmaceutically acceptable carrier.

In another embodiment the disclosed antimicrobial peptides may be used as a food preservative or in treating food products to control, reduce, or eliminate potential pathogens or contaminants. A peptide of the invention may be used as a disinfectant, for use in or with any product that must remain microbial free or be within certain tolerances. In an embodiment, treatment with a peptide provides at least partial regulation of infection or contamination.

In another embodiment it is also possible to incorporate or distribute the antimicrobial peptides within materials, on devices, or on objects (e.g. on an accessible surface), where microbial growth or viable presence is undesirable, as a method of microbicidal or microbistatic inhibition of microbial growth by administering to the devices or objects a microbicidal or microbistatic effective amount of peptide. In an embodiment, such devices or objects include, but are not limited to, linens, cloth, plastics, latex fabrics, natural rubbers, implantable devices, surfaces, or storage containers.

In another embodiment the disclosure provides methods for disinfecting a surface of an article, said method comprising the step of applying to said surface an effective amount of a composition comprising at least one microbial peptide of the invention. In an embodiment, the invention provides a disinfecting solution comprising at least one microbial peptide of the invention and optionally an acceptable carrier.

As shown below in Scheme I, two series of peptidopolysaccharides were synthesized to study the effect of lysine (K) and phenylalanine (F), which contain cationic hydrophilic and hydrophobic amino acid residues, respectively: (1) chitosan-*graft*-polylysine series (denoted CS-*g*-Kₙ) and (2) chitosan-*graft*-poly(lysine-*ran*-phenylalanine) series (denoted CS-*g*-KₙFₙ).

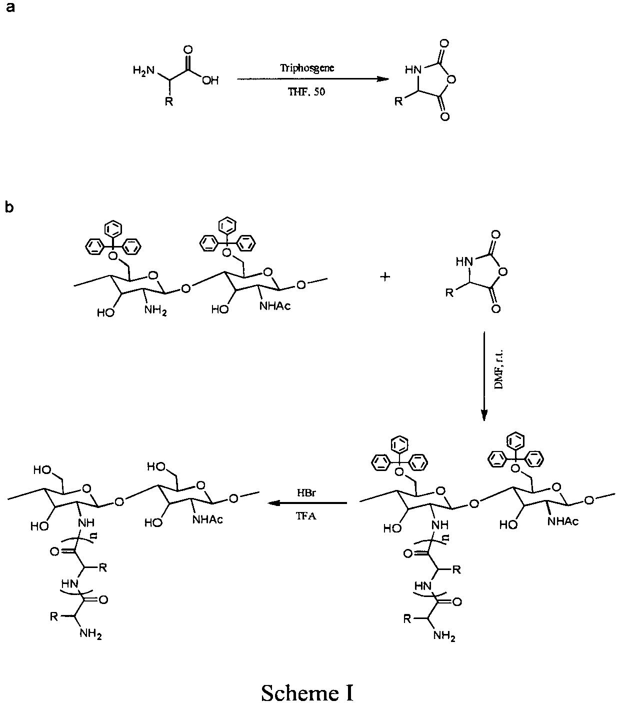

Scheme I shows the synthetic preparation of: (a) α-amino NCA monomer; and (b) Chitosan-*g*-NCA copolymers. As shown above Scheme I (a), an α-amino N-carboxyanhydride monomer may be prepared from the corresponding amino acid and triphosgene. Then, as shown in Scheme 1(b), the peptide side chains made from lysine and phenylalanine may be grafted onto the backbone of chitosan by an α-amino acid N-carboxyanhydride ring-opening polymerization (NCA ROP) initiated by the free amino groups. N-carboxyanhydride ring-opening polymerization is a facile, inexpensive and easily scalable synthetic technique. For most samples, the chitosan backbone used has a relatively low molecular weight (4.5 × 10⁴ g mol⁻¹, unless otherwise stated). A higher molecular weight (1.1 × 10⁶ g mol⁻¹) chitosan sample may also be used for studying the effect of molecular weight. Protection of the 6-CH₂OH groups on chitosan using a protecting group, for example, a triphenylmethyl group and the like, allows the amine group at the C-2 position of chitosan to react with an α-amino acid N-carboxyanhydride monomer, so that the polypeptide side chains grow from the chitosan main chain. Deprotection of the 6-CH₂OH group under acidic conditions (HBR/TFA) provided the desired AMPs.

The grafting ratio (molar ratio of the grafted amino acid units in the side chains per chitosan macromolecule) in these compounds may be varied to exploit their effect on antimicrobial activity. The synthesis of chitosan-*graft*-polypeptide copolymers were confirmed by ¹H NMR analyses. The grafting ratios were determined by the integrals of polypeptide and saccharide ¹H NMR signals. The copolymers were named by the grafting ratio shown in Table I. The number-average degree of polymerization (DPₙ), which represents the average length of the polypeptide side chain, can be calculated using the grafting ratio divided by the deacetylation degree of chitosan. The molecular weights and polydispersities determined by gas phase chromatography (GPC) are also summarized in Table I.

**Table I: Grafting Ratios**

| Copolymers | Grafting ratio | DPₙ | *Mn* (× 10⁴ g mol⁻¹) | *Mw* (× 10⁴ g mol⁻¹) | PDI |
|---|---|---|---|---|---|
| CS-*g*-K₁ | 1.1 | 1.2 | 1.26 | 1.53 | 1.22 |
| CS-*g*-K₃ | 3.4 | 3.8 | 1.35 | 1.57 | 1.16 |
| CS-*g*-K₉ | 9.0 | 10 | 2.04 | 2.32 | 1.14 |
| CS-*g*-K₁6 | 16.9 | 18.8 | 2.17 | 2.46 | 1.13 |
| CS-*g*-K₂₅ | 23.7 | 26.3 | 3.13 | 3.38 | 1.08 |
| CS-*g*-K₃-HMW | 3.1 | 3.9 | 29.2 | 33.9 | 1.16 |

| | | | | | |
|---|---|---|---|---|---|
| DPₙ (number-average degree of polymerization) = grafting ratio/deacetylation degree. *Mₙ*: number-average molecular weight. *M_{w}*: weight-average molecular weight. PDI: polydispersity index. | | | | | |

Thus, in one embodiment the disclosure provides a new class of antimicrobial polymer based cationic peptidopolysaccharides, specifically a copolymer of chitosan and polylysine, which is bacterial cell wall peptidoglycan mimetic.

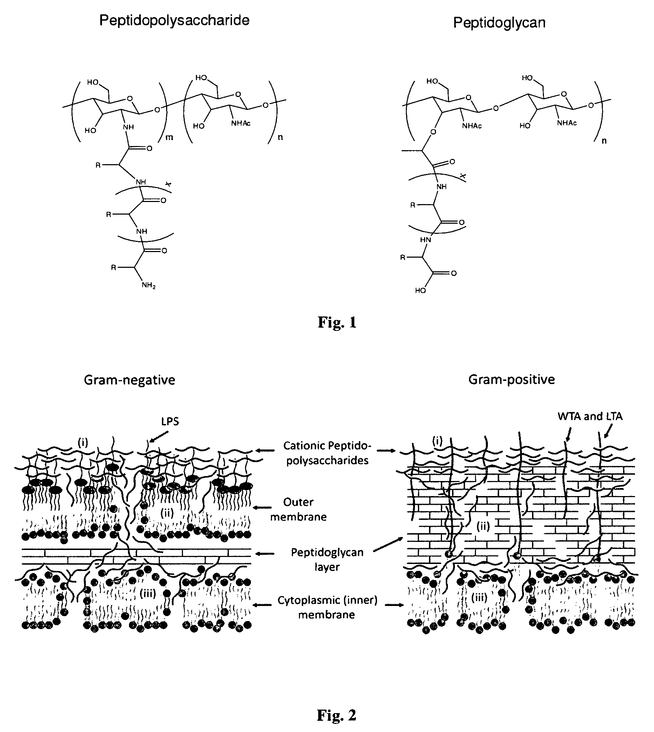

Figure 1 shows the chemical structure of a cationic peptidopolysaccharide (chitosan-*graft*-polypeptide) (R = Lys or Phe); and bacterial cell wall peptidoglycan (R = Ala, Glu, Lys, *etc*.).

Figure 2 illustrates the proposed mode of action for these cationic peptidopolysaccharides on Gram-negative and Gram-positive bacteria. As shown in Figure 2(i), the cationic peptidopolysaccharide aggregates on the cell wall by electrostatic interaction with the anionic bacterial cell wall. In Figure 2(ii), the compatibility of the cationic peptidopolysaccharides with the lipopolysaccharides (LPS) and the peptidoglycans in the cell wall allows the cationic peptidopolysaccharides to pass through the outer membrane and peptidoglycan layer. Finally, in Figure 2(iii), the cationic peptidopolysaccharides accumulate in the anionic cytoplasmic membrane of the cell wall, permeating and then disintegrating the lipid bi-layer, leading to the lethal stage of destruction of the microbe.

The cationic chitosan-*graft*-polylysine has excellent broad-spectrum antimicrobial properties against clinically significant Gram-negative (*Escherichia coli* (ATCC8739), *Pseudomonas aeruginosa* (ATCC9027)) and Gram-positive (*Staphylococcus aureus* (ATCC6538)) bacteria and fungi (*Candida albicans* (ATCC10231), *Fusarium solani* (ATCC36031)) as demonstrated by its low minimum inhibitory concentrations (5-20 µg ml⁻¹), their high selectivity for these pathogenic cells over human red blood cells (> 5-10 K) and low toxicity to mammalian cells.

The antimicrobial activity of synthetic polymers that mimic natural AMPs is known to be affected by two major factors, namely their cationic charge and their amphillic nature (hydrophobicity). Two series of chitosan-*graft*-polypeptides were synthesized and tested for their antimicrobial and hemolytic activities. As shown below in Table II, Series 1 (#1A - #1E) chitosan-*graft*-polypeptide includes polylysine with varying grafting ratios from 1 to 25 and hence, varying cationic charges. In addition, Series 2 (#2A - #2B) chitosan-*graft-*polypeptide includes poly(lysine-*ran*-phenylalanine) with equimolar hydrophobic phenylalanine and cationic lysine residues having grafting ratios of 16 (#2A) and 25 (#2B). Both Series 1 and 2 were found to be water soluble at neutral pH.

**Table II: Antimicrobial and Hemolytic Activities**

| No. | Family | Name | MIC (µg ml⁻¹) | | | | | HC₅₀ (µg ml⁻¹) | Selectivity |
|---|---|---|---|---|---|---|---|---|---|
| | | | Gram-Negative | | Gram-Positive | Fungi | | | |
| | | | *P. aeruginosa* | *E. coli* | *S. aureus* | *C. albicans* | *F solani* | | |
| 1A | Peptidopolysaccharides | CS-*g*-K₁ | 80 | 80 | 160 | 40 | 40 | >50000 | >625 |
| 1B | Peptidopolysaccharides | CS-*g*-K₃ | 20 | 20 | 40 | 20 | 10 | >50000 | >2500 |
| 1C | Peptidopolysaccharides | CS-*g*-K₉ | 20 | 10 | 20 | 20 | 5 | >50000 | >5000 |
| 1D | Peptidopolysaccharides | CS-*g*-K₁ | 20 | 10 | 10 | 20 | 5 | >10000 0 | >10000 |
| 1E | Peptidopolysaccharides | CS-*g*-K₂₅ | 40 | 10 | 10 | 20 | 5 | >50000 | >5000 |
| 2A | Peptidopolysaccharides | CS-*g*-K₈F₈ | >2500 | >2500 | 2500 | >2500 | 160 | 12500 | <5 |
| 2B | Peptidopolysaccharides | CS-*g-*K_{12.5}F_{12.5} | 1250 | 630 | 1250 | 310 | 80 | 7500 | 12 |
| 1B-HWM | Peptidopolysaccharides | CS-*g*-K₃-HMW | 310 | 80 | 160 | 160 | 40 | >25000 | >313 |
| 3A | Quaternized chitosan | TMC-Q-*g*EM | 98 | 200 | 98 | -- | 24 | >25000 | >125 |
| 3B | Quaternized chitosan | DMDC-Q-*g*EM | 98 | 98 | 49 | -- | 24 | >25000 | >250 |
| 4A | Natural AMPs | Magainin-2 | - | 125 | | -- | | >250 | >2 |
| 4B | Natural AMPs | Melittin | - | 13 | | -- | | 2 | 0.15 |
| 5A | Synthetic AMPs | P(K₂₅) | >1000 | >1000 | >1000 | >1000 | | -- | -- |
| 5B | Synthetic AMPs | P(K_{12.5}F_{12.5}) | 250 | 62 | 31 | 250 | | 16 | 0.5 |
| 5C | Synthetic AMPs | P(K₁₀F₁₅) | 31 | 31 | 31 | 125 | | 16 | 0.5 |
| 5D | Synthetic AMPs | 1-Pro₆⁹ | -- | 5 | -- | -- | | 170 | 34 |
| 5E | Synthetic AMPs | (LLRR)₄ | -- | -- | -- | >50 | | 12 | <0.5 |
| 5F | Synthetic AMPs | H1 | 32 | 8 | 32 | -- | | 128 | 16 |
| 6A | Synthetic Polymers | Polyvinylpri dine P(2VP-*co*-PEGMA 300)-HB | -- | 1.4 | -- | | | 1 | 0.71 |
| 6B | Synthetic Polymers | Polymethacr ylate PB₂₇ | -- | 16 | -- | | | 13 | 0.81 |
| 6C | Synthetic Polymers | Poly(β-lactam) *p-*(*tert-*Bu)-benzoyl | -- | 6.25 | 6.25 | | | 106 | 17 |
| 6D | Synthetic Polymers | Polynorbom ene Poly3_{0.8}-*co*-PolyP_{0.2} | -- | 15 | 25 | | | <50 | <3.3 |
| 6E | Synthetic Polymers | Pyridinium methacrylate copolymer B₄ | -- | 15 | -- | | | 0.23 | 0.02 |

| | | | | | | | | | |
|---|---|---|---|---|---|---|---|---|---|
| HC₅₀: the concentration which is 50% hemolytic; Selectivity: HC₅₀/MIC(*E*. *coli)* MIC: Minimal Inhibitory Concentration | | | | | | | | | |

In Series 1, for CS-g-Kₙ (# 1A to # 1E) with polylysine grafts of varying average lengths, the antimicrobial activities improved significantly as the polylysine ratio increased from 1 to 9 (# 1A to # 1C), with MICs of 5-20 µg ml⁻¹ for # 1C for all pathogens tested. The MICs vary only slightly between n = 9, 16, and 25 (#1C-1E), with the minimum MICs of the CS-g-Kₙ series exhibited by CS-*g*-K₁₆ (#1D). The optimum CS-g-K₁₆ (#1D) MICs are better than those of known highly effective AMPs such as magainin and mellitin (#4A, #4B, Table 1). The CS-*g*-Kₙ series (#1) has much lower MICs than the corresponding cationic homopolymer made by the same N-carboxyanhydride ring-opening polymerization method described above, but without the chitosan backbone; for example, P(K₂₅) (#5A) did not show significant antimicrobial activity whilst CS-g-K₂₅ (# 1E) has a low MIC.

In Series 2, CS-g-K₈F₈ and CS-g-K_{12.5}F_{12.5} (#2A, #2B) have much higher MICs, particularly against the bacteria. CS-g-K_{12.5}F_{12.5} (#2B) has a MIC of 630 µg ml⁻¹ and higher against the bacterial pathogens. Chitosan with a shorter poly(lysine-*ran*-phenylalanine) graft, CS-g-K₈F₈ (#2A), shows an even higher MIC of 2500 µg ml⁻¹ or greater. These two Series 2 members, *i.e.* CS-g-K₈F₈ and CS-g-K_{12.5}F_{12.5}, show relatively good antifungal activities despite their poor antibacterial activities.

Also compared are the MICs of CS-*g*-KₙFₙ (Series 2) with the corresponding values for the polymeric form of the KₙFₙ graft (#5B): the MICs of CS-*g*-K_{12.5}F_{12.5} (#2B) increased more than 5 times for the three bacteria compared with P(K_{12.5}F)_{12.5}) (#5B), which has a low MIC for both Gram-negative and Gram-positive bacteria (62 µg ml⁻¹ for *E. coli,* 31 µg ml⁻¹ for *S. aureus*).

Previous work indicated that the copeptide of KₙFₙ containing both hydrophobic and cationic amino acid residues (*e.g*. #5B) has better antimicrobial activities than the homopeptide of K₂ₙ (*e.g.* #5A) due to the requirements of hydrophobicity and cationic charge in antimicrobial biomacromolecules. By contrast, the current results are astonishing in that the Series 1 compounds have excellent antimicrobial activities in spite of the absence of hydrophobic phenylalanine residues, and CS-g-K₁₆ and CS-g-K₂₅ (#1D, #1E) show much better antimicrobial activities than CS-g-K₈F₈ and CS-*g*-K_{12.5}F_{12.5} (#2A, #2B).

Among the tested peptidopolysaccharide copolymers, CS-g-K₁₆ (#1D) has the best antimicrobial activity. It appears that the graft of pure polylysine results in better antimicrobial activity than the graft comprising the copeptide of lysine and phenylalanine. It is also apparent that the chitosan backbone significantly promotes the antimicrobial activity of the graft copolymer. The hydrophobicity of chitosan backbone and the cationic charge of lysine are hypothesized to contribute to the increased efficacy of the graft copolymer. Also, it appears that the antimicrobial activity saturates at high polylysine loading of the copolymer: there is a plateau of very low MIC between the grafting ratio of 9 and 25, with CS-g-K₁₆ being slightly better than the other two.

The molecular weight of the synthetic antimicrobial polymers is another important factor in tuning the efficacy of these polymers. The 2 Series discussed thus far, *i.e.* CS-g-Kₙ and CS-*g*-KₙFₙ (#1A to #1E and #2A - #2B), used chitosan with a relatively low molecular weight of 4.5 × 10⁴ g mol⁻¹. A higher molecular weight chitosan (1.1 × 10⁶ g mol⁻¹ in #1B-HMW) was also used to exploit the effect of molecular weight on antimicrobial activities. After the multiple reaction steps, the number-average molecular weight (*Mn*) of CS-*g*-K₃ and CS-*g*-K₃-HMW (#1B, #1B-HMW, Table II) determined by gas phase chromatography (GPC) were 1.35 ×10⁴ g mol⁻¹ and 29.2 × 10⁴ g mol⁻¹ respectively.

At the same grafting ratio of lysine, the lower molecular weight CS-*g*-K₃ shows a much lower MIC against all five pathogens. The MICs of CS-g-K₃-HMW (#1B-HMW) are 4- or more-fold as large as those of CS-*g*-K₃ (#1B), indicating that the lower molecular weight chitosan results in more effective antimicrobial graft copolymers.

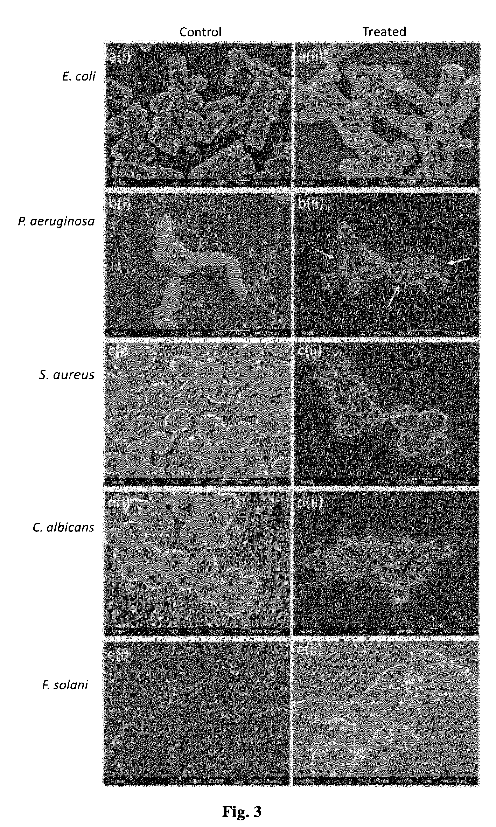

Figure 3 shows the morphology of various pathogens cells treated with CS-*g*-K₁₆ using Field Emission Scanning Electron Microscopy (FESEM). The microbial cells treated with cationic peptidopolysaccharide (CS-g-K₁₆ at MIC concentration) exhibit obvious morphological changes compared with the intact controls. Unlike the smooth surfaces of the control cells, wrinkled cell walls can be seen on *E. coli, S. aureus* and *C*. *albicans* cells. Some *P. aeruginosa* cell membranes were damaged to the point that their cell contents leaked out (see, arrows in Figure 3b(ii)), while whole cells were collapsed for *S. aureus* and *C*. *albicans. F. solani* cells surface were wrinkled and many blebs (white dots) were observed on the surface. All these results indicate that CS-*g*-K₁₆ caused severe changes in the microbe cells.

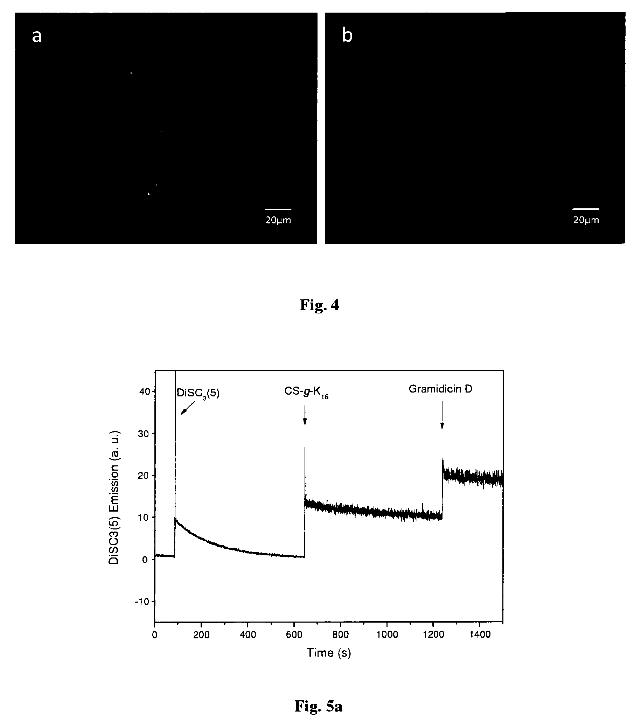

Figure 4 shows a bacterial viability study by LIVE/DEAD stain of *E. coli*, the intact bacteria fluoresce green while cationic peptidopolysaccharide (CS-*g*-K₁₆ at MIC concentration) treated ones fluoresce red. The green color dye SYTO 9 enters both intact and membrane-compromised cells, while the red color propidium iodide dye can only enter membrane damaged cells, and reduces the green SYTO dye. The observed fluoresce behavior indicates that CS-*g*-K₁₆ renders *E.coli* cell membrane permeable to propidium iodide. Moreover, unlike the intact *E. coli,* the dead cells aggregated together after treatment with CS-*g*-K₁₆.

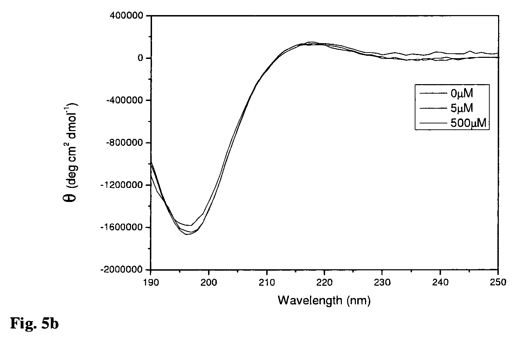

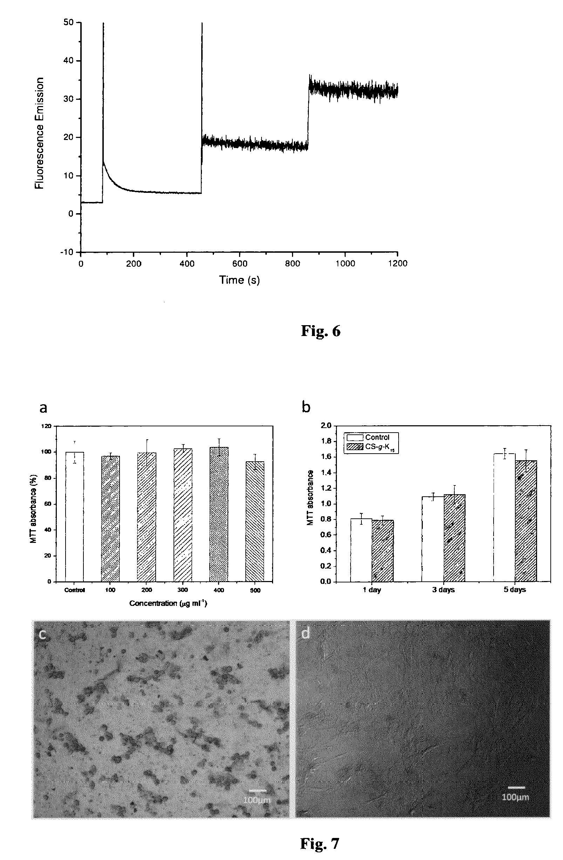

Figure 5 illustrates the microbe membrane disruption by CS-*g*-K₁₆ by adding the membrane potential sensitive dye DiSC₃(5) which is absorbed into intact membrane bilayers and is released upon subsequent membrane disruption. In Figure 5(a), the first peak indicates the addition of DiSC₃(5) and the gradual incorporation into Gram-negative bacteria *E. coli* membrane. Addition of CS-*g*-K₁₆ at the concentration of 100 µg ml⁻¹ (at around 650 s), results in an immediate fluorescence increase which indicates release of the dye due to cell membrane disruption. The fluorescence peak at around 1250s is due to 100% leakage of the dye by gramidicin D. Together with the FESEM morphology study and LIVE/DEAD assay, this result provides strong evidence that CS-*g*-K₁₆ acts to disrupt and/or permeabilize the bacterial membrane. Figure 6 demonstrates similar results of the membrane potential sensitive dye DiSC₃(5) leakage on Gram-positive bacteria *S. aureus* challenged with CS-*g-*K₁₆.

The antimicrobial activity of natural AMPs has generally been attributed to their secondary structures such as α-helix or β-sheet which promote their interaction with the bacterial membrane. Various theories for the action mechanism of α-helical or β-sheet AMPs such as the "barrel-stave", "carpet" and "toroidal" models have been proposed. Circular dichroism (CD) analysis was carried out to assess the degree to which secondary structure might contribute to the antimicrobial activity of CS-*g*-K₁₆. Figure 5(b) shows the CD spectra of CS-*g*-K₁₆ in sodium phosphate buffer with 0 µM, 5 µM, 500 µM small unilamellar liposomes (POPC/POPG 4:1). The POPC/POPG lipid vesicle is a mimic of anionic bacterial membranes. Whether in the presence or absence of the liposomes, CS-*g*-K₁₆ gives a typical random coil spectrum. Therefore, unlike most natural AMPs, these cationic peptidopolysaccharides exert their antimicrobial activity in the random coil conformation. The possibility that secondary structures may be unnecessary for good antimicrobial activity has also recently been proposed by others.

Bacterial cell walls are generally negatively charged, regardless of their specific structure and composition. The Gram-negative bacteria cell wall has two concentric lipid bilayer membranes: the cytoplasmic (inner) membrane and the outer membrane. The outer leaflet of the outer membrane of Gram-negative bacteria is mainly composed of lipopolysaccharides (LPS), which are highly negatively charged. The cytoplasmic membrane consists of anionic phospholipids together with zwitterionic phospholipids. The Gram-positive bacteria typically possess a single anionic membrane (the cytoplasmic membrane), and a thick exposed peptidoglycan layer which is rich in anionic teichoic acids (wall teichoic acid (WTA) and lipoteichoic acid (LTA)). When bacteria, whether Gram-negative or Gram-positive, come into contact with cationic peptidopolysaccharides, an electrostatic interaction between the negatively charged cell wall (due to LPS or teichoic acid) and the positively charged peptidopolysaccharides is inevitable.

While not wishing to be bound by theory, the following antibacterial mode of action for the disclosed cationic peptidopolysaccharides is proposed as shown in Figure 1(b). First, in close proximity to the microbe surface, the cationic peptidopolysaccharide is electrostatically attracted into contact with the anionic microbe cell wall, specifically by the LPS or teichoic acid in Gram-negative and Gram-positive bacteria, respectively. The negatively charged bacterial cell wall, which is stabilized by divalent cations, serves as an effective permeability barrier to restrict molecular traffic into and out of the cell. The binding of cationic peptidopolysaccharides with negative charged cell wall components (such as LPS or teichoic acids) may affect the other cationic ions such as Mg²⁺ and Ca²⁺ present in the cell wall, alter bacterial surface morphology and increase membrane permeability and diminish its barrier function.

Next, as the cationic peptidopolysaccharide molecules are attracted to and aggregated on/in the cell wall, increasing its permeability, their compatibility with LPS and peptidoglycan which also contain polysaccharides, enables them to pass through the outer membrane and the peptidoglycan layer and then reach the cytoplasmic membrane. The structural similarity of the disclosed cationic antimicrobial polymers with the exposed microbe cell wall is a unique feature not previously explored.

Finally, after passing through the bacterial cell wall barrier, cationic peptidopolysaccharides reach the cytoplasmic membrane and accumulate on/in the cytoplasmic membrane like a "carpet", permeating and then disintegrating the bi-layer when a threshold concentration is reached, leading to the lethal stage. Because of their random coil structure, it may be postulated that it is more likely that the cationic peptidopolysaccharides exert their antimicrobial activity by a "carpet model" mechanism which does not require specific structure.

The morphology and membrane depolarization results support the proposed mechanism. The FESEM images exhibit morphology changes of wrinkled or lysed cell walls (Figure 2). The DiSC₃(5) dye, which incorporates into the cytoplasmic membrane, was released after the addition of CS-*g*-K₁₆, which indicates that the cationic peptidopolysaccharides traversed the outer membrane or peptidoglycan matrix successfully and permeated the inner membrane (see, Figure 5a and Figure 6).

This proposed mechanism can also account for many features of the MIC data. It was observed that at low lysine loading (*e.g.* with CS-*g*-K₁ and CS-*g*-K₃), the CS-*g*-Kₙ series is more effective against Gram-negative bacteria than Gram-positive bacteria because the former bacterial cell wall is more anionic and hydrophilic than that of Gram-positive bacteria. The low lysine loading molecules are more strongly electrostatically attracted to Gram-negative than to Gram-positive cell walls. At high lysine loading, the attraction is strong for both types of bacteria, so that the differential attraction is less obvious and the antimicrobial activity saturates against both types of bacteria, with similar low MICs. Also, among molecules with similar compositional affinity to cell wall constituents but with different sizes, higher molecular weight molecules should less readily pass through the cell wall, resulting in reduced antimicrobial efficacy. Precisely this effect is observed in the diminished efficacy of the cationic peptidopolysaccharide made with high molecular weight chitosan (#1B-HMW) in comparison with its low MW counterpart with the same length peptide grafts (#1B).

The disclosed cationic peptidopolysaccharides have antifungal activities that are comparable to (in the case of *F. solani*, better than) their antibacterial activities. The fungal cytoplasmic membrane is anionic and protected by a polysaccharide (mainly chitin, the origin source of chitosan) cell wall. As with bacteria, the cationic peptidopolysaccharides are able, due their structural affinity to cell wall components, to pass through the polysaccharide fungal cell wall and disrupt the anionic lipid bi-layer, resulting in cell death. The collapse of *C. albicans* cells and the wrinkling of the surface of *F. solani* cells exposed to CS-*g*-K₁₆ (Figure 2d(ii), e(ii)) suggest severe disruption of the fungal cell wall and membrane.

It was also found that Series 1 exhibits extremely low hemolytic activity: the 50% hemolytic concentration (HC₅₀) for all agents in this series is greater than 50,000 µg ml⁻¹ (Table II). For CS-g-K₁₆, which has the lowest MICs, even at the highest tested concentration of 100,000 µg ml⁻¹, the hemolytic percentage is below 50% (Table II). The HC₅₀ of CS-*g*-K₁₆ is thousands of times its MICs for all tested bacteria and fungi, indicating an extremely high degree of selectivity for microbes over human red blood cells. The selectivity of CS-*g*-K₁₆ for *E. coli* over human blood red cells is more than 10,000, which is dramatically superior to the selectivity of the related P(K₁₀F₁₅) polypeptide (Table II) also synthesized by NCA ROP and is the highest ever reported.

The biocompatibility of CS-*g**-K₁₆ was also tested by MTT cell viability assay and the co-culture of mammalian Smooth Muscle Cells (SMCs) and pathogens in the presence and absence of the cationic peptidopolysaccharides. Figure 7(a) shows SMCs viability as determined by the MTT method after exposure to different concentration of CS-*g*-K₁₆ for 1 h. There is no significant decrease in SMCs survival below 500 µg ml⁻¹ of CS-*g*-K₁₆. SMCs proliferation in a culture medium supplemented with 100 µg ml⁻¹ CS-*g*-K₁₆ is statistically similar to that of the control from 1 to 5 days (Figure 7(b), *p* > 0.05, no significant difference). In co-culture of SMCs and *E. coli* bacteria, CS-*g*-K₁₆ inhibits the bacterial growth but permits SMCs growth. Figures 7(c) and 7(d) show SMCs cultured 6 h after inoculation of 1 × 10⁵ *E. coli* in culture medium without any antibiotic and with 100 µg ml⁻¹ CS-*g*-K₁₆ respectively. The presence of bacteria interfered with SMCs growth obviously (Figure 7(c)): the SMCs detached from the culture plate and have a round shape. In the 100 µg ml⁻¹ CS-*g-*K₁₆ culture system, the high concentration (10 times MIC) of CS-*g*-K₁₆ destroyed all the *E. coli* and the SMCs spread and attached to the culture substrate and showed a normal spindle shape.

Although various synthetic polymers have been designed, only a few of them show potent antimicrobial activity together with non-hemolysis. Most of them mimic the natural AMP structures that typically segregate into distinct cationic and hydrophobic domains or phases. While the antimicrobial activity can be improved significantly by optimizing the cationic/hydrophobic ratio, the hemolysis or cytotoxicity generally is also increased by this optimization. Various optimized synthetic polymers have been shown to have impressive MICs but all are rather hemolytic (#5D to #6E, Table 1). The disclosed cationic peptidopolysaccharides show an optimized MIC with CS-*g*-K₁₆ in the concentration range of 5 - 20 µg ml⁻¹, which is comparable to the best reported antimicrobial polymers (#5D to #6E, Table 1). However, CS-*g*-K₁₆ shows extraordinarily low hemolysis - HC₅₀ values that are 3 to 5 orders of magnitude larger - and excellent biocompatibility compared with other state-of-the-art antimicrobial polymers.

Unlike bacterial and fungal cells, the outer leaflet of mammalian cell membrane consists of zwitterionic phospholipids and cholesterol, which makes the net charge neutral. With mammalian cell membranes, the absence of negatively charged targets for cationic antimicrobial molecules makes their hydrophobicity the determinant of their disruption and permeabilization. The chitosan backbone is generally thought to be hydrophobic and is a well known non-toxic and low hemolysis material. The disclosed cationic peptidopolysaccharides have a hydrophobic core flanked by cationic side branches; in the absence of anionic membrane components which would attract the molecule into close contact with the membrane, the hydrophilic branches covering the chitosan hydrophobic core makes them unlikely to penetrate into the hydrophobic interior of an uncharged mammalian lipid bilayer, while the anionic charges in bacterial bilayer make such penetration more likely. Therefore, cationic peptidopolysaccharides like CS-*g*-K₁₆ show excellent non-hemolysis and biocompatibility as well as potent antimicrobial activity.

The disclosed chitosan-*graft*-polylysine, a kind of cationic peptidopolysaccharide, shows outstanding broad spectrum activities against clinically significant bacteria and fungi and record high selectivity of the pathogens over mammalian red blood cells. These cationic peptidopolysaccharides were prepared facilely by NCA ring-opening copolymerization of α-amino acids on chitosan. It has been found that CS-*g*-K₁₆ is particularly effective against both Gram-negative and Gram-positive bacteria and against fungi with low MICs (5 - 20 µg ml⁻¹) but has extremely high HC₅₀ of > 100,000 µg ml⁻¹. It is also shown that CS-*g*-K₁₆ disrupts the bacterial membrane causing death. Interestingly, antimicrobial activity may be optimized with the CS-*g*-Kₙ series with grafts containing only cationic residues (lysine), without the presence of hydrophobic residues (phenylalanine). The high antimicrobial efficacy and selectivity of the disclosed CS-*g*-Kₙ series is possibly due to the resemblance with the bacterial cell wall peptidoglycan but lack of peptidoglycan and charge in mammalian cell membrane respectively. The excellent broad-spectrum antimicrobial activities and high selectivity make cationic peptidopolysaccharides a promising prospect in antimicrobial applications.

### EXAMPLES

The following examples are provided in order to demonstrate and further illustrate certain preferred embodiments and aspects of the present invention and are not to be construed as limiting the scope thereof.

### Materials

Chitosan low molecular weight (4.5 × 10⁴ g mol⁻¹, 90% deacetylated) and high molecular weights (1.1 × 10⁶ g mol⁻¹, 80% deacetylated), Nε-benzyloxycarbonyl-L-lysine, L-phenylalanine, triphosgene, tetrahydrofuran (THF), Phthalic anhydride, triphenylchloromethane, hydrazine solution, hydrogen bromide (33% in acetic acid), N, N-dimethylformamide (DMF), pyridine, trifluoracetic acid (TFA), sodium hydroxide (NaOH), sodium phosphate dibasic, sodium phosphate monobasic, 2-mercaptoethanol, 3-(4,5-dimethylthiazol-2-yl)-2,5-diphenyltetrazolium bromide (MTT), dimethyl sulfoxide (DMSO), gramicidin D, glutaraldehyde, and osmium tetroxide were purchased from Sigma-Aldrich Corp. (St Louis, US). Tris buffer was purchased from Vivantis Biochemical Company (Subang Jaya, Malaysia). LIVE/DEAD BacLight bacterial viability kit L13152 was purchased from Invitrogen (Carlsbad, US). All bacteria and fungi strains were obtained from ATCC: Escherichia coli (ATCC8739), Pseudomonas aeruginosa (ATCC9027), Staphylococcus aureus (ATCC6538), Candida albicans (ATCC10231), and Fusarium solani (ATCC36031). All broth and agar were purchased from Becton Dickinson Company (Franklin Lakes, US).

### Synthesis of NCA Monomers

The NCA monomers were synthesized by the methods known in the art. Briefly, 10 g of the amino acid was suspended in 100 mL of anhydrous THF and the mixture was heated to 50°C, followed by addition of one-third equivalent amount of triphosgene and stirring for 3h. The reaction mixture was then filtered and the filtrate was poured into 300 mL hexane and stored at -20 °C for 24 h to fully precipitate the product. The recrystallized NCA monomer was then filtered and washed with hexane, and vacuum dried at 50 °C overnight.

### Synthesis of 6-O-Triphenylmethyl Chitosan

6-O-triphenylmethyl chitosan was prepared by a method similar to that reported by Yu et al.

N-Phthaloyl Chitosan: 5g Chitosan was dispersed in 100ml anhydrous DMF and stirred at 80 °C for 1h in order to completely dissolve it. The solution was reacted with 13.8 g phthalic anhydride (93 mmol) at 130 °C for 12h under Ar protection. After this reaction the product was precipitated in 500 ml ethanol and filtered. The product was washed with water, ethanol and diethyl ether consecutively, and dried under vacuum.

N-Phthaloyl-6-O-Triphenylmethyl Chitosan: 5g N-phthaloyl chitosan was dissolved in 100ml anhydrous pyridine then added with 15 g (55 mmol) triphenylchloromethane. The mixture was stirred at 90 °C for 24 h under Ar protection, after which the solvent was evaporated under vacuum and the product was washed with ethanol and diethyl ether consecutively.

6-O-Triphenylmethyl Chitosan: 5 g N-Phthaloyl-6-O-triphenylmethyl chitosan were added in 100 ml 50%wt hydrazine solution and stirred at 100 °C for 18 h under Ar protection. Then the suspension was filtered and the residue was washed with water, ethanol and diethyl ether consecutively.

### Synthesis of Chitosan-g-NCA Copolymers

Chitosan-g-NCA copolymers were prepared by a method similar to that reported by Yu et al. The 6-O-triphenylmethyl chitosan was dispersed in and NCA monomer was dissolved in 50 ml anhydrous DMF and stirred at room temperature for 5 d, after which the product was precipitated into acetone and dried in vacuum. To obtain the final copolymers, 6-O-triphenylmethyl chitosan-g-NCA copolymers were deprotected with HBr. Typically, 1.0g of the copolymer was dissolved in 15 ml TFA, 10 ml of HBr (33% in acetic acid) was added and the mixture was stirred for 4 h in an ice bath. The product was precipitated with acetone, then filtered and dissolved in distilled water, and the pH was adjusted to 7 with NaOH (1M). After that the product was dialyzed with cellulose membrane (Sigma, M. W. 10334) and then freeze-dried.

### Characterization of Polymers

NMR spectra of the synthesized products were obtained with a Bruker Avance DPX 300 instrument. The molecular weights and polydispersity were measured using a Shimadzu LC-20AD instrument equipped with a refractive index detector (RID), using a PL-aquagel-OH GPC column and sodium acetate buffer (0.2 M, pH 4.05). Samples were analyzed at 40°C with an eluent flow rate of 1.0 ml min⁻¹.

### Minimal Inhibitory Concentrations (MICs):

Bacteria cells were grown overnight at 37 °C in MH broth to a mid-log phase and diluted to 10⁴ to 10⁵ CFU ml⁻¹. A twofold dilution series of 100µl product solution in MH broth (YM broth for the fungi C. albicans and F.solani) was made on 96-well microplate, followed by the addition of 100µl bacterial suspension (10⁴ to 10⁵ CFU mL⁻¹). The plates were incubated at 37 °C for 18-24h (28 °C, 36-48h for fungi), and the absorbance at 600nm was measured with a microplate reader (BIO-RAD, US). Positive control was without product, and negative control was without bacteria/fungi inoculum. MICs were determined as the lowest concentration that inhibited cell growth by more than 90%.

### Morphology Study of Microorganisms:

The morphology changes of microorganisms induced by CS-g-K₁₆ were examined with FE-SEM after 15 min-treatment with CS-g-K₁₆ at MIC concentration. After incubation the microbes were immediately fixed with glutaraldehyde (2.5%, 5 ml) solution for 4 hr. Following dehydration in a graded ethanol series (20%-100%) and drying under a nitrogen flow, the samples were coated with platinum and imaged with FESEM (JEOL JSM-6700F) for microbe morphology changes.

### LIVE/DEAD Assay to Examine Bacterial Viability

LIVE/DEAD assay was carried out using CS-g-K₁₆ with E. coli. A suspension of E. coli (10⁸ CFU ml⁻¹) in PBS was prepared followed by addition of CS-g-K₁₆ at MIC concentration; the mixture was incubated at 37 °C for 15 min. After incubation the cells were collected by centrifugation and stained with BacLight bacterial viability kit L13152 (Invitrogen) for 30 min at room temperature. The cells were then imaged with fluorescence microscopy (Carl Zeiss, Germany).

### Membrane Depolarization:

Bacteria were harvested at mid-log phase, washed three times with a buffer solution of 20 mM glucose and 5 mM HEPES (pH 7.4). The bacteria was resuspended in the washing buffer with additional 0.1 M KCl, then incubated with DiSC₃(5) (20 nM) until it was incorporated into the cell membrane. The CS-g-K₁₆ was added at a concentration of 100 µg ml⁻¹, and the fluorescence was monitored with a fluorescence spectrometer (PerkinElmer LS55, U.S.) at λₑₓ=620 nm and λₑₘ=670 nm. Finally, 0.2 nM Gramicidin D was added to obtain the full release of the dye.

### Circular Dichroism (CD)

The POPC:POPG (molar ratio, 4:1) small unilamellar liposomes were prepared using a mini-extruder (Avanti Polar Lipids-Mini Extruder, U.S.). 50 µg ml⁻¹ CS-g-K₁₆ was dissolved in 20 mM sodium phosphate buffer (pH 7.4) and CD spectra were measured with the addition of 0, 5 and 500 µM liposomes at room temperature, using a circular dichroism spectrometer (Chirascan^{™}, Applied Photophysics, UK).

### Hemolysis Assay

Fresh human blood (5 ml) was collected from a healthy donor (age 25, Male). Erythrocytes were separated by centrifugation at 1000 rpm for 10 min, washed three times with Tris buffer (10 mM Tris, 150 mM NaCl, pH 7.2) and diluted to final concentration (5%, v/v). 50µl of antimicrobial solution at different concentrations mixed with 50µl erythrocytes stock were added to a 96-well microplate and incubated for 1 h at 37 °C with 150 rpm shaking. The microplate were centrifuged at 1,000 rpm for 10 min. 80 µl aliquots of the supernatant were then transferred to a new 96-well microplate and diluted with another 80 µl of Tris buffer. Hemolysis was measured as the absorbance at 540 nm with a microplate reader (BIO-RAD, US). Complete hemolysis was determined in Tris buffer plus 0.1% Triton x-100 as positive control, while Tris buffer was used as negative control. The percentage of hemolysis (H) was calculated using the following equation: Hemolysis = [(Oₚ - O_{b})/(Oₜ -O_{b})] x100%, where Oₚ is the density for a given antimicrobial agent concentration, O_{b} is the density for the buffer, and Oₜ is the density for the Triton x-100 positive control.

### In Vitro Biocompatibility Studies

The biocompatibility study was carried out with smooth muscle cells (SMCs). SMCs were cultured in 96-well plates from initial inocula of 1 × 10⁵ cells in each well. A graded concentration series of CS-g-K₁₆ solutions in culture medium was prepared, 200 µl of which was added to the SMC cultures. The cells were incubated at 37 °C for the desired time, then the culture medium with CS-g-K₁₆ was changed with fresh DMEM and 20 µl of MTT solution (5 mg ml⁻¹ in PBS) was added. After another 4 h incubation, the MTT medium was removed. 100 µl DMSO was added and the plate was shaken at 100 rpm for 15 min. The cell viability was measured by the absorbance of each well at 490 nm.

### Co-Culture of Bacteria and Mammalian Cells

SMCs were cultured in 96-well plates from inocula of 1 × 10⁵ cells in each well and incubated at 37 °C for 24 h in a culture medium free of any antibiotics. 1 × 10⁵ cells E.coli was seeded in the SMC culture wells. Then CS-g-K₁₆ was added in the SMC and E. coli co-culture wells at a concentration of 100 µg ml⁻¹. Wells without CS-g-K₁₆ were used as a control. The co-culture was imaged using an inverted optical microscope (Carl Zeiss, Germany).

### References:

M. A. Fischbach, C. T. Walsh, Science 2009, 325, 1089.
C. J. Waschinski, J. Zimmermann, U. Salz, R. Hutzler, G. Sadowski, J. C. Tiller, Advanced Materials 2008, 20, 104.
D. A. Salick, D. J. Pochan, J. P. Schneider, Advanced Materials 2009, 21, 4120.
R. E. W. Hancock, H. G. Sahl, Nature Biotechnology 2006, 24, 1551.
M. Zasloff, Nature 2002, 415, 389.
N. Papo, Y. Shai, Peptides 2003, 24, 1693.
N. Y. Yount, A. S. Bayer, Y. Q. Xiong, M. R. Yeaman, Biopolymers 2006, 84, 435.
I. S. Radzishevsky, S. Rotem, D. Bourdetsky, S. Navon-Venezia, Y. Carmeli, A. Mor, Nature Biotechnology 2007, 25, 657.
N. P. Chongsiriwatana, J. A. Patch, A. M. Czyzewski, M. T. Dohm, A. Ivankin, D. Gidalevitz, R. N. Zuckermann, A. E. Barron, Proceedings of the National Academy of Sciences of the United States of America 2008, 105, 2794.
G. N. Tew, R. W. Scott, M. L. Klein, W. F. Degrado, Accounts of Chemical Research 2010, 43, 30.
M. R. Yeaman, N. Y. Yount, Pharmacological Reviews 2003, 55, 27.
K. Lienkamp, G. N. Tew, Chemistry-a European Journal 2009, 15, 11784.
C. C. Zhou, X. B. Qi, P. Li, W. N. Chen, L. Mouad, M. W. Chang, S. S. J. Leong, M. B. Chan-Park, Biomacromolecules 2010, 11, 60.
A. C. Engler, A. Shukla, S. Puranam, H. G. Buss, N. Jreige, P. T. Hammond, Biomacromolecules 2011, 12, 1666.
I. Sovadinova, E. F. Palermo, R. Huang, L. M. Thoma, K. Kuroda, Biomacromolecules 2011, 12, 260.
T. R. Stratton, B. M. Applegate, J. P. Youngblood, Biomacromolecules 2011, 12, 50.
K. Lienkamp, A. E. Madkour, A. Musante, C. F. Nelson, K. Nusslein, G. N. Tew, Journal of the American Chemical Society 2008, 130, 9836.
P. Li, Y. F. Poon, W. Li, H. Y. Zhu, S. H. Yeap, Y. Cao, X. Qi, C. Zhou, M. Lamrani, R. W. Beuerman, E. T. Kang, Y. Mu, C. M. Li, M. W. Chang, S. S. Jan Leong, M. B. Chan-Park, Nature Materials 2011, 10, 149.
G. J. Gabriel, A. Som, A. E. Madkour, T. Eren, G. N. Tew, Materials Science & Engineering R-Reports 2007, 57, 28.
H. J. Yu, X. S. Chen, T. C. Lu, J. Sun, H. Y. Tian, J. Hu, Y. Wang, P. B. Zhang, X. B. Jing, Biomacromolecules 2007, 8, 1425.
T. J. Deming, Prog. Polym. Sci. 2007, 32, 858.
M. F. Ilker, K. Nusslein, G. N. Tew, E. B. Coughlin, Journal of the American Chemical Society 2004, 126, 15870.
Z. M. Al-Badri, A. Som, S. Lyon, C. F. Nelson, K. Nusslein, G. N. Tew, Biomacromolecules 2008, 9, 2805.
E. Glukhov, L. L. Burrows, C. M. Deber, Biopolymers 2008, 89, 360.
V. Sambhy, B. R. Peterson, A. Sen, Angewandte Chemie-International Edition 2008, 47, 1250.
K. Kuroda, G. A. Caputo, W. F. DeGrado, Chemistry-a European Journal 2009, 15, 1123.
B. P. Mowery, A. H. Lindner, B. Weisblum, S. S. Stahl, S. H. Gellman, Journal of the American Chemical Society 2009, 131, 9735.
E. F. Palermo, K. Kuroda, Biomacromolecules 2009, 10, 1416.
G. Cabrini, A. S. Verkman, Journal of Membrane Biology 1986, 92, 171.
K. A. Brogden, Nature Reviews Microbiology 2005, 3, 238.
T. J. Beveridge, Journal of Bacteriology 1999, 181, 4725.
W. Fischer, Medical Microbiology and Immunology 1994, 183, 61.
R. E. W. Hancock, Trends in Microbiology 1997, 5, 37.
Y. Shai, Biopolymers 2002, 66, 236.
Y. C. Chung, Y. P. Su, C. C. Chen, G. Jia, H. I. Wang, J. C. G. Wu, J. G. Lin, Acta Pharmacologica Sinica 2004, 25, 932.
T. Theis, U. Stahl, Cellular and Molecular Life Sciences 2004, 61, 437.
N. Wiradharma, U. Khoe, C. A. E. Hauser, S. V. Seow, S. G. Zhang, Y. Y. Yang, Biomaterials 2011, 32, 2204.
H. T. Chou, T. Y. Kuo, J. C. Chiang, M. J. Pei, W. T. Yang, H. C. Yu, S. B. Lin, W. J. Chen, International Journal of Antimicrobial Agents 2008, 32, 130.
S. Colak, C. F. Nelson, K. Nusslein, G. N. Tew, Biomacromolecules 2009, 10, 353.
M. Rinaudo, Prog. Polym. Sci. 2006, 31, 603.
J. C. Fernandes, P. Eaton, H. Nascimento, L. Belo, S. Rocha, R. Vitorino, F. Amado, J. Gomes, A. Santos-Silva, M. E. Pintado, F. X. Malcata, Biomacromolecules 2008, 9, 3346.
D. Kahne, C. Leimkuhler, L. Wei, C. Walsh, Chemical Reviews 2005, 105, 425.
H. J. Yu, W. S. Wang, X. S. Chen, C. Deng, X. B. Jing, Biopolymers 2006, 83, 233.

Although the disclosure has been described with reference to the above examples, it will be understood that the disclosure is limited only by the following claims.

## Claims

1. A compound of Formula I: 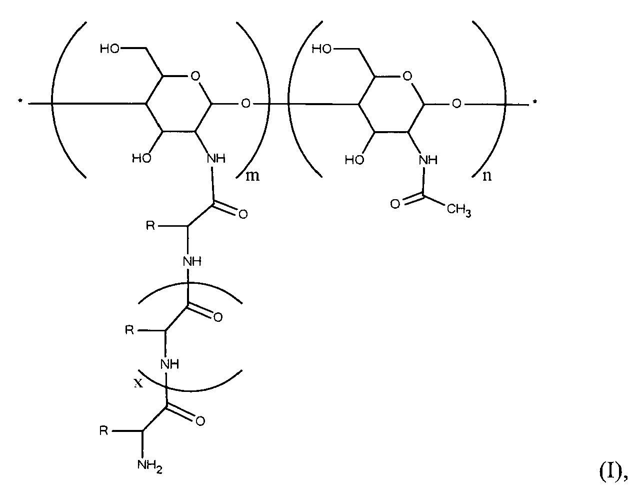 or a pharmaceutically acceptable salt thereof, wherein:
each R is independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, (CH₂)ⱼOR¹, (CH₂)ᵢC(O)R¹, (CH₂)ⱼC(O)OR¹, (CH₂)ⱼOC(O)R¹, (CH₂)ⱼNR²R³, (CH₂)ⱼC(O)NR²R³, (CH₂)ⱼOC(O)NR²R³, (CH₂₎ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ and (CH₂)ⱼNR⁴S(O)ₖNR²R³, wherein j is an integer independently selected from 0 to 6, k is an integer independently selected from 0 to 2, and wherein each R group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
R¹, R², R³ and R⁴ are each independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, wherein each R¹, R², R³ and R⁴ group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
m is an integer independently selected from 1 to 100,000;
n is an integer independently selected from 1 to 100,000; and
x is an integer independently selected from 1 to 5,000.

2. The compound of claim 1, wherein:
R is independently selected from substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, substituted or unsubstituted (C₃-C₁₀)heteroaryl(C₁-C₆)alkyl, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R⁴, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ and (CH₂)NR⁴S(O)ₖNR²R³; and
R¹, R², R³ and R⁴ are each independently selected from hydrogen, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, and substituted or
unsubstituted (C₃-C₁₀)heteroaryl(C₁-C₆)alkyl.

3. The compound of claim 1 or 2, wherein:
R is independently selected from (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, hetero(C₃-C₁₀)aryl, hetero(C₃-C₁₀)aryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR⁴S(O)ₖCH₃, S(O)ₖNR²R³ and NR⁴S(O)ₖNR²R³; and
R¹, R², R³ and R⁴ are each independently selected from hydrogen, (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, (C₃-C₁₀)heteroaryl, and (C₃-C₁₀)heteroaryl(C₁-C₆)alkyl.

4. The compound of any of claims 1 to 3, wherein R is independently selected from CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆Hs, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH and CH₂SCH₃.

5. The compound of any of claims 1 to 4, wherein the compound of Formula I is selected from:

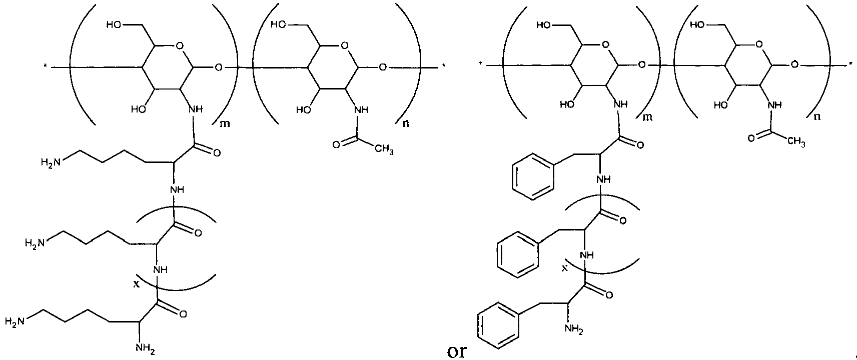

6. A pharmaceutical composition comprising a compound of Formula I of any of claims 1 to 5, and a pharmaceutically acceptable carrier.

7. The pharmaceutical composition of claim 6 for controlling infection by a microorganism.

8. The pharmaceutical composition of claim 7 for controlling infection by a microorganism, wherein the microorganism is selected from a bacterium, a fungus, a virus, and a protozoan.

9. The pharmaceutical composition of claim 8 for controlling infection by a microrganism, wherein the microorganism is selected from a gram-negative bacterium and a gram-positive bacterium.

10. An ex-vivo method of disinfecting a surface of an article, the method comprising the step of applying to the surface an effective amount of a composition, wherein the composition comprises the compound of Formula I of any of claims 1 to 5.

11. A disinfecting solution comprising the compound of Formula I of any of claims 1 to 5, and optionally an acceptable carrier.

12. A method of preparing a compound of Formula I: 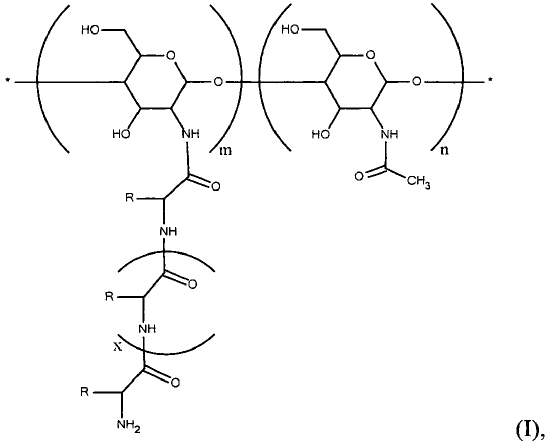 or a pharmaceutically acceptable salt thereof, the method comprising the steps of:
a) reacting a compound of Formula II with a compound of Formula III to provide a compound of Formula IV; and
b) deprotecting the compound of Formula IV to provide the compound of Formula I: 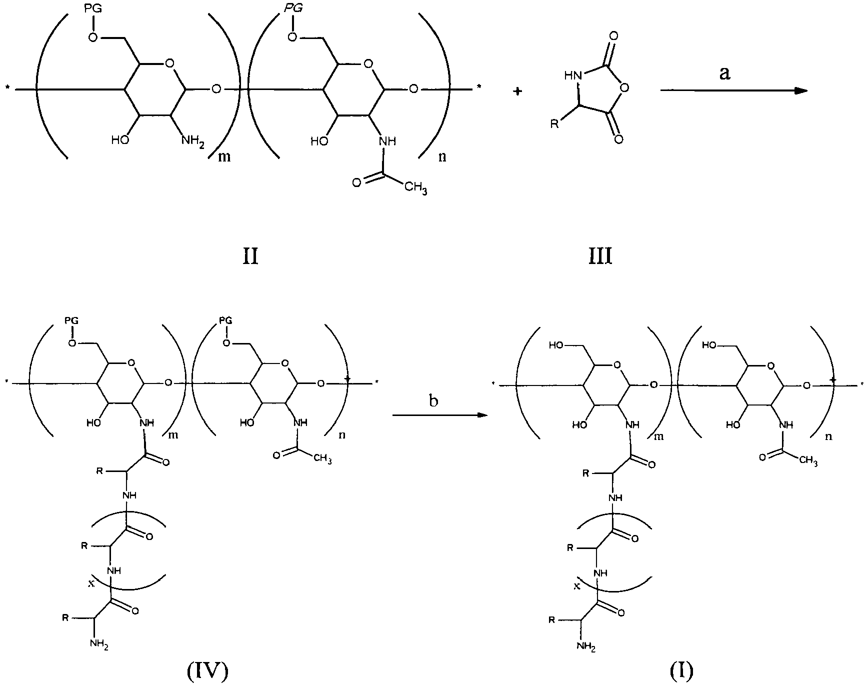 wherein:
each PG is a protecting group;
each R is independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, (CH₂)ⱼOR¹, (CH₂)ⱼC(O)R¹, (CH₂)ⱼC(O)OR¹, (CH₂)ⱼOC(O)R¹, (CH₂)ⱼNR²R³, (CH₂)ⱼC(O)NR²R³, (CH₂)ⱼOC(O)NR²R³, (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)jS(O)ₖCH₃, (CH₂)ⱼNR⁴S(O)ₖCH₃, (CH₂)ⱼS(O)ₖNR²R³ and (CH₂)ⱼNR⁴S(O)ₖNR²R³, wherein j is an integer independently selected from 0 to 6, k is an integer independently selected from 0 to 2, and wherein each R group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
R¹, R², R³ and R⁴ are each independently selected from hydrogen, substituted or unsubstituted alkyl, substituted or unsubstituted alkenyl, substituted or unsubstituted alkynyl, substituted or unsubstituted cycloalkyl, substituted or unsubstituted aryl, substituted or unsubstituted arylalkyl, substituted or unsubstituted heteroaryl, substituted or unsubstituted heteroarylalkyl, wherein each R¹, R², R³ and R⁴ group is optionally independently substituted with 1-3 substituent groups, each substituent group is independently selected from alkyl, alkenyl, alkynyl, cycloalkyl, perfluoroalkyl, amino, cyano, halogen, hydroxyl, nitro, aryl, arylalkyl, heterocycle, heteroaryl, and heteroarylalkyl;
m is an integer independently selected from 1 to 100,000;
n is an integer independently selected from 1 to 100,000; and
x is an integer independently selected from 1 to 5,000.

13. The method of claim 12, wherein:
PG is a triphenylmethyl protecting group;
R is independently selected from substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, substituted or unsubstituted (C₃-C₁₀)heteroaryl(C₁-C₆)alkyl, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ and (CH₂)NR⁴S(O)ₖNR²R³; and
R¹, R², R³ and R⁴ are each independently selected from hydrogen, substituted or unsubstituted (C₁-C₆)alkyl, substituted or unsubstituted (C₂-C₆)alkenyl, substituted or unsubstituted (C₂-C₆)alkynyl, substituted or unsubstituted (C₃-C₈)cycloalkyl, substituted or unsubstituted (C₆-C₁₀)aryl, substituted or unsubstituted (C₆-C₁₀)aryl(C₁-C₆)alkyl, substituted or unsubstituted (C₃-C₁₀)heteroaryl, and substituted or unsubstituted (C₃-C₁₀)heteroaryl(C₁-C₆)alkyl.

14. The method of claim 12 or 13, wherein:
R is independently selected from (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, hetero(C₃-C₁₀)aryl, hetero(C₃-C₁₀)aryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR⁴S(O)ₖCH₃, S(O)ₖNR²R³ and NR⁴S(O)ₖNR²R³; and
R¹, R², R³ and R⁴ are each independently selected from hydrogen, (C₁-C₆)alkyl, (C₂-C₆)alkenyl, (C₂-C₆)alkynyl, (C₃-C₈)cycloalkyl, (C₆-C₁₀)aryl, (C₆-C₁₀)aryl(C₁-C₆)alkyl, (C₃-C₁₀)heteroaryl, and (C₃-C₁₀)heteroaryl(C₁-C₆)alkyl.

15. The method of any of claims 12 to 14, wherein R is independently selected from CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆Hs, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH and CH₂SCH₃.

## Patentansprüche

1. Verbindung nach Formel I: 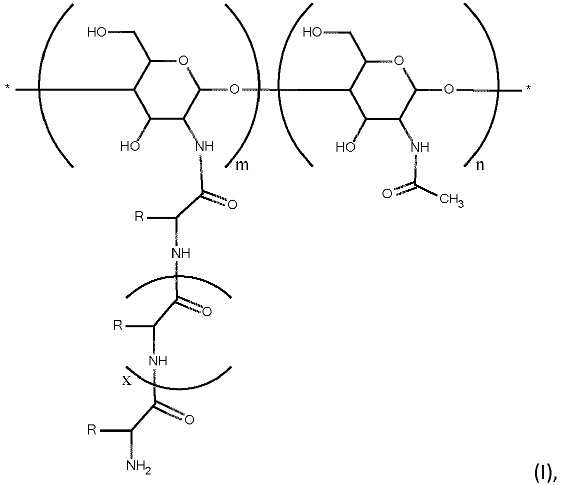 oder ein pharmazeutisch akzeptables Salz davon, wobei:
jedes R unabhängig ausgewählt ist aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, (CH₂)ⱼOR¹, (CH₂)ⱼC(O)R¹, (CH₂)ⱼC(O)OR¹, (CH₂)ⱼOC(O)R¹, (CH₂)ⱼNR²R³, (CH₂)ⱼC(O)NR²R³ (CH₂)ⱼOC(O)NR²R³ (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ und (CH₂)ⱼNR⁴S(O)ₖNR²R³, wobei j eine ganze Zahl unabhängig ausgewählt aus 0 bis 6 ist, k eine ganze Zahl unabhängig ausgewählt aus 0 bis 2, und wobei jede R-Gruppe gegebenenfalls unabhängig mit 1 bis 3 Substituentengruppen substituiert ist, jede Substituentengruppe unabhängig ausgewählt ist aus Alkyl, Alkenyl, Alkinyl, Cycloalkyl, Perfluoralkyl, Amino, Cyano, Halogen, Hydroxyl, Nitro, Aryl, Arylalkyl, Heterozyklus, Heteroaryl, und Heteroarylalkyl;
R¹, R², R³ und R⁴ sind jeweils unabhängig ausgewählt aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, wobei jede R¹, R², R³ und R⁴ -Gruppe gegebenenfalls unabhängig substituiert mit 1 bis 3 Substituentengruppen ist, jede Substituentengruppe unabhängig ausgewählt ist aus Alkyl, Alkenyl, Alkinyl, Cycloalkyl, Perfluoralkyl, Amino, Cyano, Halogen, Hydroxyl, Nitro, Aryl, Arylalkyl, Heterozyklus, Heteroaryl, und Heteroarylalkyl;
m eine ganze Zahl unabhängig ausgewählt aus 1 bis 100000 ist;
n eine ganze Zahl unabhängig ausgewählt aus 1 bis 100000 ist; und
x eine ganze Zahl unabhängig ausgewählt aus 1 bis 5000 ist.

2. Verbindung nach Anspruch 1, wobei:
R unabhängig ausgewählt ist aus substituiertem oder unsubstituiertem (C₁-C₆)Alkyl, substituiertem oder unsubstituiertem (C₂-C₆)Alkenyl, substituiertem oder unsubstituiertem (C₂-C₆)Alkinyl, substituiertem oder unsubstituiertem (C₃-C₈)Cycloalkyl, substituiertem oder unsubstituiertem (C₆-C₁₀)Aryl, substituiertem oder unsubstituiertem (C₆-C₁₀)Aryl(C₁-C₆)alkyl, substituiertem oder unsubstituiertem (C₃-C₁₀)Heteroaryl, substituiertem oder unsubstituiertem (C₃-C₁₀)Heteroaryl(C₁-C₆)alkyl, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ und (CH₂)NR⁴S(O)ₖNR²R³; und
R¹, R², R³ und R⁴ sind jeweils unabhängig ausgewählt aus Wasserstoff, substituiertem oder unsubstituiertem (C₁-C₆)Alkyl, substituiertem oder unsubstituiertem (C₂-C₆)Alkenyl, substituiertem oder unsubstituiertem (C₂-C₆)Alkinyl, substituiertem oder unsubstituiertem (C₃-C₈)Cycloalkyl, substituiertem oder unsubstituiertem (C₆-C₁₀)Aryl, substituiertem oder unsubstituiertem (C₆-C₁₀)Aryl(C₁-C₆)alkyl, substituiertem oder unsubstituiertem (C₃-C₁₀)Heteroaryl, und substituiertem oder unsubstituiertem (C₃-C₁₀)Heteroaryl(C₁-C₆)alkyl.

3. Verbindung nach Anspruch 1 oder 2, wobei:
R unabhängig ausgewählt ist aus (C₁-C₆)Alkyl, (C₂-C₆)Alkenyl, (C₂-C₆)Alkinyl, (C₃-C₈)Cycloalkyl, (C₆-C₁₀)Aryl, (C₆-C₁₀)Aryl(C₁-C₆)alkyl, (C₃-C₁₀)Heteroaryl, (C₃-C₁₀)Heteroaryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³ NR⁴C(NH)NR²R³, SH S(O)ₖCH₃, NR⁴S(O)ₖCH₃, S(O)ₖNR²R³ und NR⁴S(O)ₖNR²R³; und
R¹, R², R³ und R⁴ sind jeweils unabhängig ausgewählt aus Wasserstoff, (C₁-C₆)Alkyl, (C₂-C₆)Alkenyl, (C₂-C₆)Alkinyl, (C₃-C₈)Cycloalkyl, (C₆-C₁₀)Aryl, (C₆-C₁₀)Aryl(C₁-C₆)alkyl, (C₃-C₁₀)Heteroaryl, und (C₃-C₁₀)Heteroaryl(C₁-C₆)alkyl.

4. Verbindung nach einem der Ansprüche 1 bis 3, wobei R unabhängig ausgewählt ist aus CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH und CH₂SCH₃.

5. Verbindung nach einem der Ansprüche 1 bis 4, wobei die Verbindung nach Formel I ausgewählt ist aus:

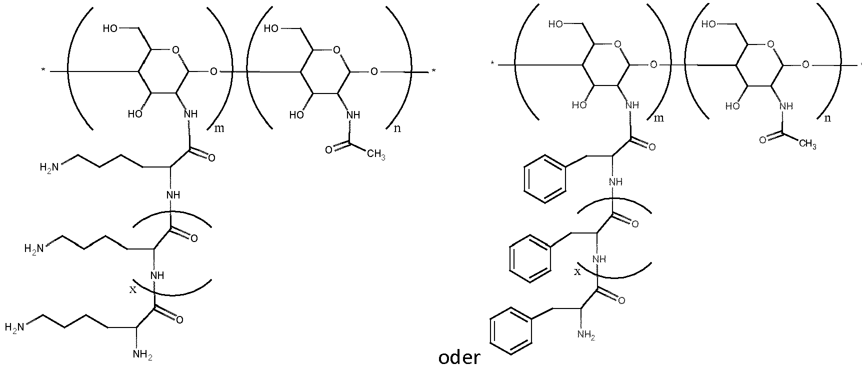

6. Pharmazeutische Zusammensetzung umfassend eine Verbindung nach Formel I nach einem der Ansprüche 1 bis 5, und einen pharmazeutisch akzeptablen Träger.

7. Pharmazeutische Zusammensetzung nach Anspruch 6 zum Kontrollieren einer Infektion durch einen Mikroorganismus.

8. Pharmazeutische Zusammensetzung nach Anspruch 7 zum Kontrollieren einer Infektion durch einen Mikroorganismus, wobei der Mikroorganismus ausgewählt ist aus einem Bakterium, einem Pilz, einem Virus, und einem Protozoon.

9. Pharmazeutische Zusammensetzung nach Anspruch 8 zum Kontrollieren einer Infektion durch einen Mikroorganismus, wobei der Mikroorganismus ausgewählt ist aus einem gram-negativen Bakterium und einem gram-positiven Bakterium.

10. Ex-vivo Verfahren zum Desinfizieren einer Oberfläche eines Gegenstands, wobei das Verfahren den Schritt des Verabreichens einer effektive Menge einer Zusammensetzung auf die Oberfläche umfasst, wobei die Zusammensetzung die Verbindung nach Formel I nach einem der Ansprüche 1 bis 5 umfasst.

11. Desinfizierende Lösung umfassend die Verbindung nach Formel I nach einem der Ansprüche 1 bis 5, und gegebenenfalls einen akzeptablen Träger.

12. Verfahren zur Herstellung einer Verbindung nach Formel I: 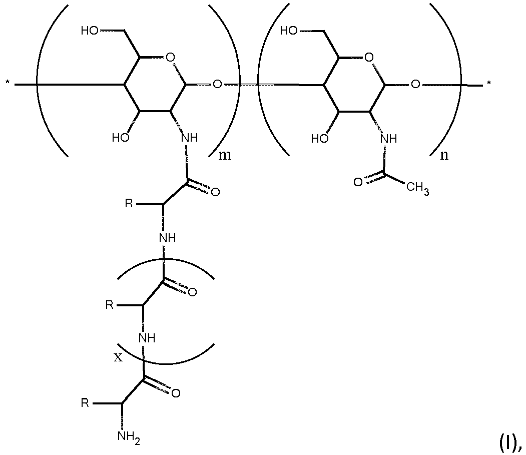 oder ein pharmazeutisch akzeptables Salz davon, wobei das Verfahren die Schritte umfasst von:
a) Umsetzen einer Verbindung nach Formel II mit einer Verbindung nach Formel III um eine Verbindung nach Formel IV bereitzustellen; und
b) Entschützen der Verbindung nach Formel IV um eine Verbindung nach Formel I bereitzustellen: 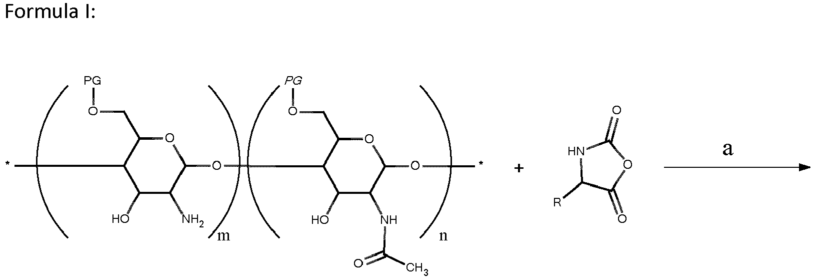 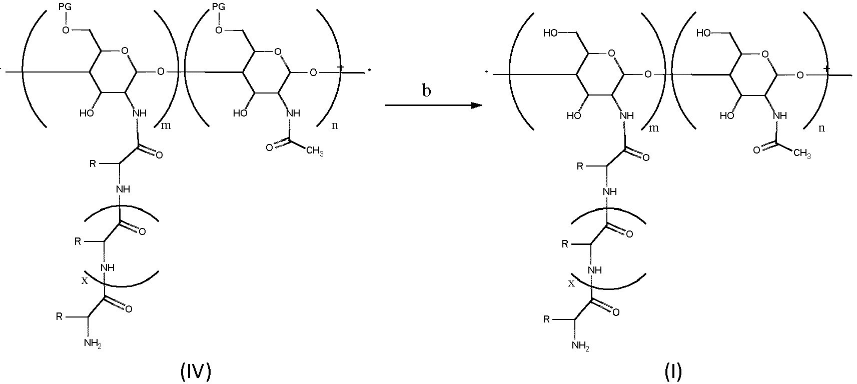 wobei:
jedes PG eine Schutzgruppe ist;
jedes R unabhängig ausgewählt ist aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, (CH₂)ⱼOR¹, (CH₂)ⱼC(O)R¹, (CH²)ⱼC(O)OR¹, (CH₂)ⱼOC(O)R¹, (CH)ⱼNR²R³ (CH)ⱼC(O)NR²R³, (CH₂)ⱼOC(O)NR²R³, (CH₂)ⱼC(NR⁴)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)jNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ und (CH₂)ⱼNR⁴S(O)ₖNR²R³, wobei j eine ganze Zahl unabhängig ausgewählt aus 0 bis 6 ist, k eine ganze Zahl unabhängig ausgewählt aus 0 bis 2, und wobei jede R-Gruppe gegebenenfalls unabhängig mit 1 bis 3 Substituentengruppen substituiert ist, jede Substituentengruppe unabhängig ausgewählt ist aus Alkyl, Alkenyl, Alkinyl, Cycloalkyl, Perfluoralkyl, Amino, Cyano, Halogen, Hydroxyl, Nitro, Aryl, Arylalkyl, Heterozyklus, Heteroaryl, und Heteroarylalkyl;
R¹, R², R³ und R⁴ sind jeweils unabhängig ausgewählt aus Wasserstoff, substituiertem oder unsubstituiertem Alkyl, substituiertem oder unsubstituiertem Alkenyl, substituiertem oder unsubstituiertem Alkinyl, substituiertem oder unsubstituiertem Cycloalkyl, substituiertem oder unsubstituiertem Aryl, substituiertem oder unsubstituiertem Arylalkyl, substituiertem oder unsubstituiertem Heteroaryl, substituiertem oder unsubstituiertem Heteroarylalkyl, wobei jede R¹, R², R³ und R⁴ -Gruppe gegebenenfalls unabhängig substituiert mit 1 bis 3 Substituentengruppen ist, jede Substituentengruppe unabhängig ausgewählt ist aus Alkyl, Alkenyl, Alkinyl, Cycloalkyl, Perfluoralkyl, Amino, Cyano, Halogen, Hydroxyl, Nitro, Aryl, Arylalkyl, Heterozyklus, Heteroaryl, und Heteroarylalkyl;
m eine ganze Zahl unabhängig ausgewählt aus 1 bis 100000 ist;
n eine ganze Zahl unabhängig ausgewählt aus 1 bis 100000 ist; und
x eine ganze Zahl unabhängig ausgewählt aus 1 bis 5000 ist.

13. Verfahren nach Anspruch 12, wobei:
PG eine Triphenylmethyl-Schutzgruppe ist;
R unabhängig ausgewählt ist aus substituiertem oder unsubstituiertem (C₁-C₆)Alkyl, substituiertem oder unsubstituiertem (C₂-C₆)Alkenyl, substituiertem oder unsubstituiertem (C₂-C₆)Alkinyl, substituiertem oder unsubstituiertem (C₃-C₈)Cycloalkyl, substituiertem oder unsubstituiertem (C₆-C₁₀)Aryl, substituiertem oder unsubstituiertem (C₆-C₁₀)Aryl(C₁-C₆)alkyl, substituiertem oder unsubstituiertem (C₃-C₁₀)Heteroaryl, substituiertem oder unsubstituiertem (C₃-C₁₀)Heteroaryl(C₁-C₆)alkyl, (CH₂)OR¹, (CH₂)CO)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹, (CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ und (CH₂)NR⁴S(O)ₖNR²R³; und
R¹, R², R³ und R⁴ sind jeweils unabhängig ausgewählt aus Wasserstoff, substituiertem oder unsubstituiertem (C₁-C₆)Alkyl, substituiertem oder unsubstituiertem (C₂-C₆)Alkenyl, substituiertem oder unsubstituiertem (C₂-C₆)Alkinyl, substituiertem oder unsubstituiertem (C₃-C₈)Cycloalkyl, substituiertem oder unsubstituiertem (C₆-C₁₀)Aryl, substituiertem oder unsubstituiertem (C₆-C₁₀)Aryl(C₁-C₆)alkyl, substituiertem oder unsubstituiertem (C₃-C₁₀)Heteroaryl, und substituiertem oder unsubstituiertem (C₃-C₁₀)Heteroaryl(C₁-C₆)alkyl.

14. Verfahren nach Anspruch 12 oder 13, wobei:
R unabhängig ausgewählt ist aus (C₁-C₆)Alkyl, (C₂-C₆)Alkenyl, (C₂-C₆)Alkinyl, (C₃-C₈)Cycloalkyl, (C₆-C₁₀)Aryl, (C₆-C₁₀)Aryl(C₁-C₆)alkyl, (C₃-C₁₀)Heteroaryl, (C₃-C₁₀)Heteroaryl(C₁-C₆)alkyl, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR⁴S(O)ₖCH₃, S(O)ₖNR²R³ und NR⁴S(O)ₖNR²R³; und
R¹, R², R³ und R⁴ sind jeweils unabhängig ausgewählt aus Wasserstoff, (C₁-C₆)Alkyl, (C₂-C₆)Alkenyl, (C₂-C₆)Alkinyl, (C₃-C₈)Cycloalkyl, (C₆-C₁₀)Aryl, (C₆-C₁₀)Aryl(C₁-C₆)alkyl, (C₃-C₁₀)Heteroaryl, und (C₃-C₁₀)Heteroaryl(C₁-C₆)alkyl.

15. Verfahren nach einem der Ansprüche 12 bis 14, wobei R unabhängig ausgewählt ist aus CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂C₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH und CH₂SCH₃.

## Revendications

1. Composé de Formule I : 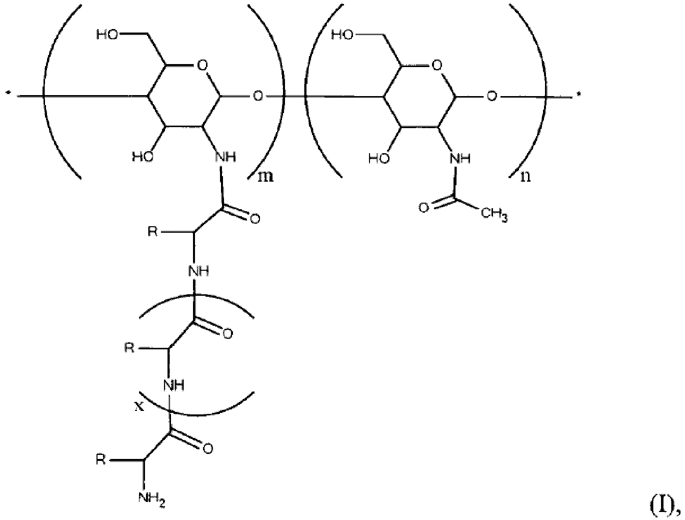 ou sel pharmaceutiquement acceptable de celui-ci, dans laquelle :
chaque R est indépendamment sélectionné parmi hydrogène, alkyle substitué ou non substitué, alkényle substitué ou non substitué, alkynyle substitué ou non substitué, cycloalkyle substitué ou non substitué, aryle substitué ou non substitué, arylalkyle substitué ou non substitué, hétéroaryle substitué ou non substitué, hétéroarylalkyle substitué ou non substitué, (CH₂)ⱼOR¹, (CH2)ⱼC(O)R¹, (CH₂)ⱼC(O)OR¹, (CH₂)ⱼOC(O)R¹, (CH₂)ⱼNR²R³, (CH₂)ⱼC(O)NR²R³, (CH₂)jOC(O)NR²R³, (CH₂)ⱼC(NR)NR²R³, (CH₂)ⱼNR⁴C(O)R¹, (CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃,
(CH₂)ⱼNR⁴S (O)ₖCH₃, (CH₂)ⱼS(O)ₖNR²R³ et (CH₂)ⱼNR⁴S(O)ₖNR²R³, dans laquelle j est un entier indépendamment sélectionné parmi 0 à 6, k est un entier indépendamment sélectionné parmi 0 à 2, et dans laquelle chaque groupe R est en option indépendamment substitué par 1 à 3 groupes substituants, chaque groupe substituant est indépendamment sélectionné parmi alkyle, alkényle, alkynyle, cycloalkyle, perfluoroalkyle, amino, cyano, halogène, hydroxyle, nitro, aryle, arylalkyle, hétérocycle, hétéroaryle et hétéroarylalkyle ;
R¹, R², R³ et R⁴ sont chacun indépendamment sélectionnés parmi hydrogène, alkyle substitué ou non substitué, alkényle substitué ou non substitué, alkynyle substitué ou non substitué, cycloalkyle substitué ou non substitué, aryle substitué ou non substitué, arylalkyle substitué ou non substitué, hétéroaryle substitué ou non substitué,
hétéroarylalkyle substitué ou non substitué, dans laquelle chaque groupe R¹, R², R³ et R⁴ est en option indépendamment substitué par 1 à 3 groupes substituants, chaque groupe substituant est indépendamment sélectionné parmi alkyle, alkényle, alkynyle, cycloalkyle, perfluoroalkyle, amino, cyano, halogène, hydroxyle, nitro, aryle, arylalkyle, hétérocycle, hétéroaryle et hétéroarylalkyle ;
m est un entier indépendamment sélectionné parmi 1 à 100 000 ;
n est un entier indépendamment sélectionné parmi 1 à 100 000 ; et
x est un entier indépendamment sélectionné parmi 1 à 5000.

2. Composé selon la revendication 1, dans lequel :
R est indépendamment sélectionné parmi (C₁-C₆)alkyle substitué ou non substitué, (C₂-C₆)alkényle substitué ou non substitué, (C₂-C₆)alkynyle substitué ou non substitué, (C₃-C₈)cycloalkyle substitué ou non substitué, (C₆-C₁₀)aryle substitué ou non substitué, (C₆-C₁₀)aryl (C₁-C₆) alkyle substitué ou non substitué, (C₃-C₁₀) hétéroaryle substitué ou non substitué, (C₃-C₁₀)hétéroaryl(C₁-C₆) alkyle substitué ou non substitué, (CH₂)OR¹, (CH₂)C(O)R¹, (CH₂)C(O)OR¹, (CH₂)OC(O)R¹, (CH₂)NR²R³, (CH₂)C(O)NR²R³, (CH₂)OC(O)NR²R³, (CH₂)C(NR⁴)NR²R³, (CH₂)NR⁴C(O)R¹, (CH₂)NR⁴C(O)OR¹,
(CH₂) NR⁴C(O)NR²R³, (CH₂) NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ et (CH₂)NR⁴S(O)ₖNR²R³ ; et
R¹, R², R³ et R⁴ sont chacun indépendamment sélectionnés parmi hydrogène, (C₁-C₆)alkyle substitué ou non substitué, (C₂-C₆)alkényle substitué ou non substitué, (C₂-C₆)alkynyle substitué ou non substitué,
(C₃-C₈)cycloalkyle substitué ou non substitué, (₆-C₁₀)aryle substitué ou non substitué, (C₆-C₁₀)aryl (C₁-C₆)alkyle substitué ou non substitué, (C₃-C₁₀)hétéroaryle substitué ou non substitué et (C₃-C₁₀)hétéroaryl(C₁-C₆) alkyle substitué ou non substitué.

3. Composé selon la revendication 1 ou 2, dans lequel :
R est indépendamment sélectionné parmi (C₁-C₆)alkyle, (C₂-C₆)alkényle, (C₂-C₆)alkynyle, (C₃-C₈)cycloalkyle, (C₆-C₁₀)aryle, (C₆-C₁₀)aryl (C₁-C₆) alkyle, hétéro (C₃-C₁₀)aryle, hétéro (C₃-C₁₀) aryl (C₁-C₆)alkyle, OR¹, C(O)R¹, C(O)OR¹, OC(O)R¹, NR²R³, C(O)NR²R³, OC(O)NR²R³, C(NR⁴)NR²R³, NR⁴C(O)R¹, NR⁴C(O)OR¹, NR⁴C(O)NR²R³, NR⁴C(O)NR²R³, NR⁴C(NH)NR²R³, SH, S(O)ₖCH₃, NR₄S(O)ₖCH₃, S(O)ₖNR²R³ et NR⁴S(O)ₖNR²R³ ; et
R¹, R², R³ et R⁴ sont chacun indépendamment sélectionnés parmi hydrogène, (C₁-C₆)alkyle, (C₂-C₆)alkényle, (C₂-C₆)alkynyle, (C₃-C₈)cycloalkyle, (C₆-C₁₀)aryle, (C₆-C₁₀)aryl (C₁-C₆) alkyle, (C₃-C₁₀) hétéroaryle et (C₃-C₁₀)hétéroaryl (C₁-C₆) alkyle.

4. Composé selon l'une quelconque des revendications 1 à 3, dans lequel R est indépendamment sélectionné parmi CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂H₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH et CH₂SCH₃.

5. Composé selon l'une quelconque des revendications 1 à 4, dans lequel le composé de Formule I est sélectionné parmi :

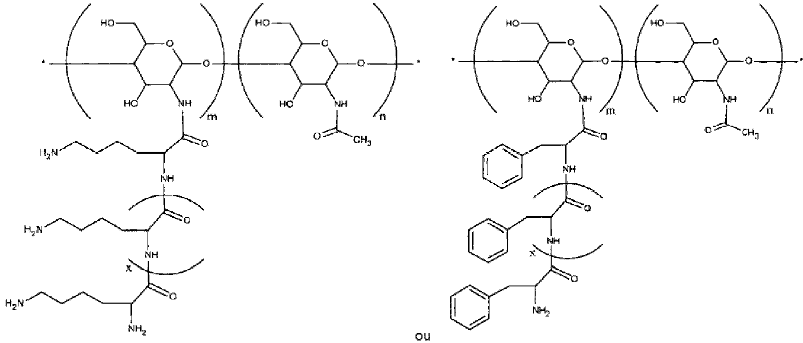

6. Composition pharmaceutique comprenant un composé de Formule I selon l'une quelconque des revendications 1 à 5 et un support pharmaceutiquement acceptable.

7. Composition pharmaceutique selon la revendication 6 pour le contrôle d'une infection par un microorganisme.

8. Composition pharmaceutique selon la revendication 7 pour le contrôle d'une infection par un microorganisme, dans laquelle le microorganisme est sélectionné parmi une bactérie, un champignon, un virus et un protozoaire.

9. Composition pharmaceutique selon la revendication 8 pour le contrôle d'une infection par un microorganisme, dans laquelle le microorganisme est sélectionné parmi une bactérie gram-négative et une bactérie gram-positive.

10. Procédé ex vivo de désinfection d'une surface d'un article, le procédé comprenant l'étape d'application à la surface d'une quantité efficace d'une composition, dans lequel la composition comprend le composé de Formule I selon l'une quelconque des revendications 1 à 5.

11. Solution désinfectante comprenant le composé de Formule I selon l'une quelconque des revendications 1 à 5 et en option un support acceptable.

12. Procédé de préparation d'un composé de Formule I : 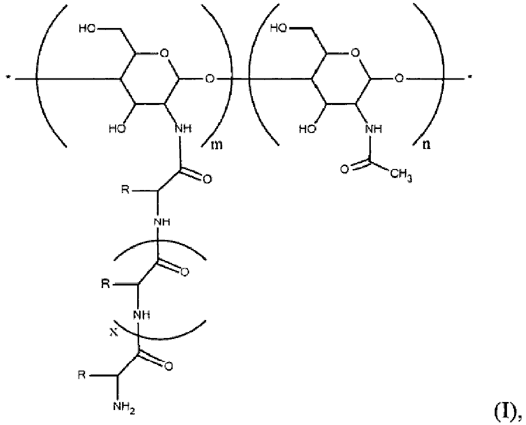 ou d'un sel pharmaceutiquement acceptable de celui-ci, le procédé comprenant les étapes consistant à :
a) faire réagir un composé de Formule II avec un composé de Formule III pour fournir un composé de Formule IV ; et
b) déprotéger le composé de Formule IV pour fournir le composé de Formule I : 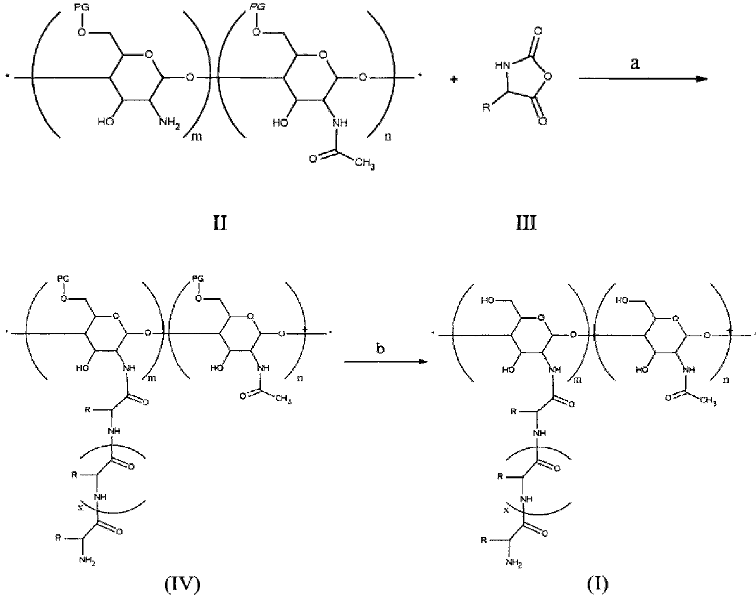
dans lesquelles :
chaque PG est un groupe protecteur ;
chaque R est indépendamment sélectionné parmi hydrogène, alkyle substitué ou non substitué, alkényle substitué ou non substitué, alkynyle substitué ou non substitué, cycloalkyle substitué ou non substitué,
aryle substitué ou non substitué, arylalkyle substitué ou non substitué, hétéroaryle substitué ou non substitué, hétéroarylalkyle substitué ou non substitué, (CH₂)ⱼOR¹, (CH2)ⱼC(O)R¹, (CH₂)ⱼC(O)OR¹, (CH₂)ⱼOC(O)R¹, (CH2)ⱼNR²R³, (CH₂)ⱼC(O)NR²R³, (CH₂)ⱼOC(O)NR²R³, (CH₂)ⱼC(NR⁴)NR²R³, (CH₂)ⱼNR⁴C(O)R¹,
(CH₂)ⱼNR⁴C(O)OR¹, (CH₂)ⱼNR⁴C(O)NR²R³, (CH₂)ⱼNR⁴C(O)NR²R³, (CH₂)ⱼNR⁴C(NH)NR²R³, (CH₂)ⱼSH, (CH₂)ⱼS(O)ₖCH₃, (CH₂)ⱼNR⁴S(O)ₖCH₃, (CH₂)ⱼS(O)ₖNR²R³ et (CH₂)ⱼNR⁴S(O)ₖNR²R³, dans lesquelles j est un entier indépendamment sélectionné parmi 0 à 6, k est un entier indépendamment sélectionné parmi 0 à 2, et dans lesquelles chaque groupe R est en option indépendamment substitué par 1 à 3 groupes substituants, chaque groupe substituant est indépendamment sélectionné parmi alkyle, alkényle, alkynyle, cycloalkyle, perfluoroalkyle, amino, cyano, halogène, hydroxyle, nitro, aryle, arylalkyle, hétérocycle, hétéroaryle et hétéroarylalkyle ;
R¹, R², R³ et R⁴ sont chacun indépendamment sélectionnés parmi hydrogène, alkyle substitué ou non substitué, alkényle substitué ou non substitué, alkynyle substitué ou non substitué, cycloalkyle substitué ou non substitué, aryle substitué ou non substitué, arylalkyle substitué ou non substitué, hétéroaryle substitué ou non substitué, hétéroarylalkyle substitué ou non substitué, dans lesquelles chaque groupe R¹, R², R³ et R⁴ est en option indépendamment substitué par 1 à 3 groupes substituants, chaque groupe substituant est indépendamment sélectionné parmi alkyle, alkényle, alkynyle, cycloalkyle, perfluoroalkyle, amino, cyano, halogène, hydroxyle, nitro, aryle, arylalkyle, hétérocycle, hétéroaryle et hétéroarylalkyle ;
m est un entier indépendamment sélectionné parmi 1 à 100 000 ;
n est un entier indépendamment sélectionné parmi 1 à 100 000 ; et
x est un entier indépendamment sélectionné parmi 1 à 5000.

13. Procédé selon la revendication 12, dans lequel :
PG est un groupe protecteur triphénylméthyle ;
R est indépendamment sélectionné parmi (C₁-C₆)alkyle substitué ou non substitué, (C₂-C₆)alkényle substitué ou non substitué, (C₂-C₆)alkynyle substitué ou non substitué, (C₃-C₈)cycloalkyle substitué ou non substitué, (C₆-C₁₀)aryle substitué ou non substitué,
(C₆-C₁₀)aryl (C₁-C₆) alkyle substitué ou non substitué,
(C₃-C₁₀) hétéroaryle substitué ou non substitué, (C₃-C₁₀)hétéroaryl(C₁-C₆) alkyle substitué ou non substitué, (CH₂)OR¹, (CH₂)CO)R¹, (CH₂)C(O)OR¹, (CH₂)OC(O)R¹, (CH₂)NR²R³, (CH₂) C(O)NR²R³, (CH₂)OC(O)NR²R³, (CH₂) C(NR⁴)NR²R³, (CH₂)NR⁴C(O)R¹, (CH₂)NR⁴C(O)OR¹,
(CH₂)NR⁴C(O)NR²R³, (CH₂)NR⁴C(O)NR²R³, (CH₂)NR⁴C(NH)NR²R³, (CH₂)SH, (CH₂)S(O)ₖCH₃, (CH₂)NR⁴S(O)ₖCH₃, (CH₂)S(O)ₖNR²R³ et (CH₂)NR⁴S(O)ₖNR²R³; et
R¹, R², R³ et R⁴ sont chacun indépendamment sélectionnés parmi hydrogène, (C₁-C₆)alkyle substitué ou non substitué, (C₂-C₆)alkényle substitué ou non substitué, (C₂-C₆)alkynyle substitué ou non substitué,
(C₃-C₈)cycloalkyle substitué ou non substitué, (C₆-C₁₀)aryle substitué ou non substitué, (C₆-C₁₀)aryl (C₁-C₆)alkyle substitué ou non substitué, (C₃-C₁₀)hétéroaryle substitué ou non substitué et (C₃-C₁₀)hétéroaryl(C₁-C₆) alkyle substitué ou non substitué.

14. Procédé selon la revendication 12 ou 13, dans lequel :
R est indépendamment sélectionné parmi (C₁-C₆)alkyle, (C₂-C₆)alkényle, (C₂-C₆)alkynyle, (C₃-C₈)cycloalkyle, (C₆-C₁₀)aryle, (C₆-C₁₀)aryl (C₁-C₆)alkyle, hétéro (C₃-C₁₀)aryle, hétéro (C₃-C₁₀) aryl (C₁-C₆) alkyle, OR¹, C(O)R¹, C(O)OR¹, OC(O)R¹, NR²R³, C(O)NR²R³, OC(O)NR²R³,
C(NR⁴)NR²R³, NR⁴C(O)R¹, NR⁴C(O)OR¹, NR⁴C(O)NR²R³,
NR⁴C(O)NR²R³, NR⁴C(NH)NR²R³, SH, S(O)ₖCH₃, NR⁴S(O)ₖCH₃,
S(O)ₖNR²R³ et NR⁴S(O)ₖNR²R³ ; et
R¹, R², R³ et R⁴ sont chacun indépendamment sélectionnés parmi hydrogène, (C₁-C₆)alkyle, (C₂-C₆)alkényle, (C₂-C₆)alkynyle, (C₃-C₈)cycloalkyle, (C₆-C₁₀)aryle, (C₆-C₁₀) aryl (C₁-C₆) alkyle, (C₃-C₁₀) hétéroaryle et (C₃-C₁₀)hétéroaryl(C₁-C₆) alkyle.

15. Procédé selon l'une quelconque des revendications 12 à 14, dans lequel R est indépendamment sélectionné parmi CH₃, CH₂CH₃, CH₂CH₂CH₃, CH(CH₃)₂, CH₂CH₂CH₂CH₃, CH(CH₃)CH₂CH₃, CH₂CH(CH₃)₂, C₆H₅, CH₂C₆H₅, CH₂H₆H₄(para-OH), CH₂C₆H₅, CH₂OH, CH₂CH(OH)CH₃, CH₂C(O)NH₂, (CH₂)₂C(O)NH₂, (CH₂)₄NH₂, (CH₂)₃NHC(NH)NH₂, CH₂(C₃H₂N₂), CH₂(C₈H₆N), CH₂SH et CH₂SCH₃.
